(19) Europäisches Patentamt
European Patent Office
Office européen des brevets

(11) Veröffentlichungsnummer: **0 106 799**
B1

(12) **EUROPÄISCHE PATENTSCHRIFT**

(45) Veröffentlichungstag der Patentschrift: **19.03.86**

(21) Anmeldenummer: **83810446.1**

(22) Anmeldetag: **03.10.83**

(51) Int. Cl.⁴: **C 07 C 69/732,** C 23 F 11/00, C 07 C 69/738, C 07 C 69/747, C 07 C 59/48, C 07 C 69/145, C 07 C 103/78, C 07 C 69/16, C 07 C 69/88, C 07 C 79/22, C 07 C 43/174 // C07C91/06, C07C79/12

(54) **Neue Phenole und ihre Herstellung.**

(30) Priorität: **07.10.82 GB 8228643**
**10.12.82 GB 8235360**

(43) Veröffentlichungstag der Anmeldung: **25.04.84 Patentblatt 84/17**

(45) Bekanntmachung des Hinweises auf die Patenterteilung: **19.03.86 Patentblatt 86/12**

(84) Benannte Vertragsstaaten: **BE DE FR GB IT**

(56) Entgegenhaltungen:
**EP - A - 0 069 066**
**EP - A - 0 069 068**

(73) Patentinhaber: **CIBA-GEIGY AG, Klybeckstrasse 141, CH-4002 Basel (CH)**

(72) Erfinder: **Howell, Frederick Harold, Dr., 27 High Bank, Atherton Lancashire M29 9HZ (GB)**

**EP 0 106 799 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

In der vorliegenen Erfindung werden neue Phenole beschrieben, eine Methode zu ihrer Herstellung und ihre Verwendung als Stabilisatoren und Korrosionsinhibitoren.

Es ist bekannt, Alkyl-substituierte Phenole als Stabilisatoren zu verwenden. Es wurden nun neue Alkyl-substituierte Phenole gefunden, die sowohl stabilisierende als auch korrosionsinhibierende Wirkung zeigen. Die Erfindung betrifft Verbindungen der Formel I

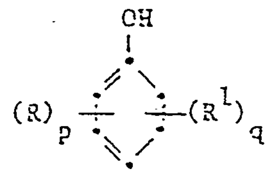

(I) ,

worin p 1,2 oder 3 ist, q 0, 1 oder 2 ist, mit der Massgabe, dass $(p+q) \leqq 3a$ ist, und worin R eine Gruppe der Formel II bedeutet

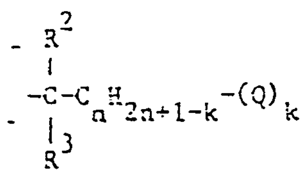

(II) ,

wobei die Gruppen R im Fall von $p = 2$ oder $p = 3$ gleich oder innerhalb der gegebenen Definitionen verschieden sind und sich in 2-, 4- oder 6-position befinden, und n eine ganze Zahl von 2 und 30 ist, und k 1 oder 2 bedeutet, und schliesslich Q einer der folgenden Reste ist:

a) $-COOR^4$ oder $-CONR^4R^5$, wobei $R^4$ Wasserstoff, geradkettiges oder verzweigtes $C_1$-$C_{20}$Alkyl, das gegebenenfalls mit 1 bis 5 Sauerstoffatomen unterbrochen ist oder gegebenenfalls Substituenten $-OR^6$ trägt, wobei $R^6$ $C_3$-$C_{12}$Cycloalkyl, geradkettiges oder verzweigtes $C_3$-$C_{20}$Alkenyl oder $C_6$-$C_{10}$Aryl bedeutet, das gegebenenfalls mit ein oder zwei $C_1$-$C_4$-Alkylgruppen substituiert ist, oder $C_7$-$C_{13}$Aralkyl ist, und wo $R^4$ zusätzlich divalentes geradkettiges oder verzweigtes $C_2$-$C_{20}$Alkylen ist, oder geradkettiges oder verzweigtes $C_3$-$C_{20}$Alkenyl bedeutet, oder $C_3$-$C_{12}$Cycloalkyl oder $C_6$-$C_{10}$Aryl bedeutet, das gegebenenfalls mit einer $C_1$-$C_4$Alkylgruppe substituiert ist, oder $C_7$-$C_{13}$Arylkyl ist, oder ein fünf- oder sechsgliedriger sauerstoffhaltiger Heterozyklus ist, der gegebenenfalls mit ein oder zwei geradkettigen oder verzweigten $C_1$-$C_4$Alkylgruppen substituiert ist, oder wo $R^4$ Methyl bedeutet, das durch einen fünf- oder sechsgliedrigen sauerstoffhaltigen gegebenenfalls ein oder zwei $C_1$-$C_4$Alkylgruppen enthaltenden Heterozyklus substituiert ist, mit der Massgabe, dass die beiden C-Atome, an denen sich die $-COOR^4$ Gruppen im Fall von $k = 2$ befinden, nicht benachbart sind, und wobei $R^5$ Wasserstoff oder geradkettiges oder verzweigtes $C_1$-$C_{20}$Alkyl ist, oder wo $R^4$ und $R^5$ zusammen mit dem gemeinsamen Stickstoffatom einen fünf- oder sechsgliedrigen heterozyklischen Ring bilden, der gegebenenfalls mit ein oder zwei $C_1$-$C_4$-Alkylgruppen substituiert sein kann,

b) -OX, wobei X die gleiche Bedeutung wie $R^5$ hat, oder $-COR^7$, wobei $R^7$ Wasserstoff, geradkettiges oder verzweigtes $C_1$-$C_{20}$Alkyl, geradkettiges oder verzweigtes $C_3$-$C_{20}$Alkenyl,$C_3$-$C_{12}$Cycloalkyl, $C_7$-$C_{13}$Aralkyl oder $C_6$-$C_{10}$Aryl ist, das gegebenenfalls mit ein oder zwei $C_1$-$C_4$Alkylgruppen substituiert sein kann,

c) $-NR^8R^9$, wobei $R^8$ Wasserstoff oder geradkettiges oder verzweigtes $C_1$-$C_4$Alkyl ist, und $R^9$ Wasserstoff, geradkettiges oder verzweigtes $C_1$-$C_4$Alkyl, oder $-COR^7$ ist, wobei $R^7$ die früher definierte Bedeutung hat, oder wobei $R^8$ und $R^9$ zusammen mit dem gemeinsamen N-Atom einen fünf- oder sechsgliedrigen Ring bilden, welcher gegebenenfalls mit ein oder zwei $C_1$-$C_4$Alkylgruppen substituiert ist,

d) $-PO(OR^{10})[O]_x$-$R^{11}$, wobei x 0 oder 1 ist, und wobei $R^{10}$und $R^{11}$ im Falle von $x = 1$ gleich oder verschieden sind und unabhängig voneinander Wasserstoff, geradkettiges oder verzweigtes $C_1$-$C_{20}$Alkyl bedeuten oder wobei $R^{10}$ und $R^{11}$ eine zusammenhängende $C_2$-$C_3$Alkylen-kette, die gegebenenfalls mit einer oder mehreren $C_1$-$C_{20}$Alkylgruppen substituiert sein kann, bilden, oder wobei im Fall $x = 0$ $R^{10}$ Wasserstoff oder geradkettiges oder verzweigtes $C_1$-$C_{20}$Alkyl ist, und $R^{11}$ geradkettiges $C_1$-$C_5$Alkyl ist,

e) -CN, Halogen, $-NO_2$ oder

f) $-COR^{12}$, wobei $R^{12}$ Wasserstoff, geradkettiges oder verzweigtes $C_1$-$C_{20}$Alkyl oder Halogen bedeutet;

und wo in Formel II $R^2$ und $R^3$ gleich oder innerhalb der gegebenen Definition verschieden sind und geradkettiges oder verzweigtes $C_1$-$C_5$-Alkyl bedeuten, oder wo $R^2$ oder $R^3$ gegebenenfalls mit einer $-COOR^4$ Gruppe substituiert ist, aber nur dann, wenn Q $-COOR^4$ ist, wobei die Reste $R^4$ voneinander unabhängig sind, oder wo $R^2$ oder $R^3$ so mit dem Rest $C_nH_{2n+1-k}$ verbunden sind, dass ein $C_5$-$C_{12}$ Cycloalkylenrest entsteht, der mit $-(CO_2R^4)_k$ Gruppen substituiert ist, wobei die Reste$R^4$ voneinander unabhängig sind und wo $R^4$ und k die

oben definierte Bedeutung haben, oder wo $R^2$ oder $R^3$ so mit dem Rest $C_nH_{2n+1-k}$verbunden sind, dass im Fall, wo $Q = -CO_2R^{12}$ ist, ein $C_5$-$C_{12}$Cycloalkylenrest entsteht, der gegebenenfalls mit einer $-COR^{12}$ Gruppe substituiert ist; und wobei im Fall, dass $Q = -COOR^4$ und $R^4 = C_2$-$C_{20}$Alkylen sind, p und k 1 bedeuten,und die Verbindung durch die Formel Ia dargestellt wird

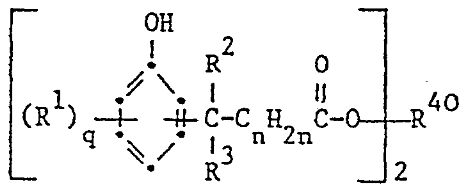

(Ia) ,

wobei $R^{40}$ eine divalente geradkettige oder verzweigte $C_2$-$C_{20}$Alkylen-kette ist;

und wobei im Fall, dass $Q = -CONR^4R^5$ und $R^4 = C_2$-$C_{20}$Alkylen sind, p und k 1 bedeuten, und die Verbindung durch die Formel 1b dargestellt wird

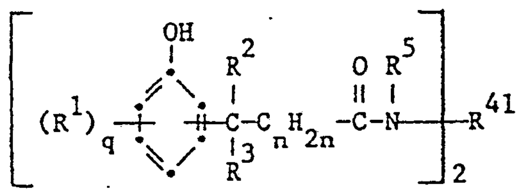

(Ib) ,

wobei $R^{41}$ ein geradkettiger oder verzweigter divalenter $C_2$-$C_{20}$Alkylenrest ist;

und wo zurückgehend auf Formel I $R^1$ geradkettiges oder verzweigtes $C_1$-$C_{12}$Alkyl, $C_7$-$C_9$Aralkyl, Halogen, $CF_3$, SH, $SR^{13}$, COOH, $COOR^{13}$, $COR^{13}$, $COC_6H_5$, $CONH_2$, CN, $SO_3H$, $SO_2NH_2$, $PO(OH)_2$, $PO(OR^{13})$, oder $NO_2$ bedeutet, wobei $R^{13}$ $C_1$-$C_4$Alkyl ist,

wobei im Fall $q = 2$ die Gruppen $R^1$ gleich oder im Rahmen der gegebenen Definitionen unterschiedlich sein können, und wobei die Gruppe $R^1$ im Fall $q = 1$ zusätzlich ein Rest der Formel III oder IV sein kann

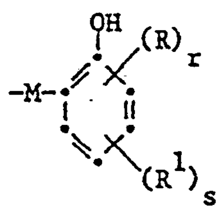

(III) ,

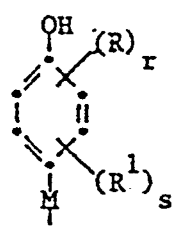

(IV) ,

worin R und $R^1$ die oben definierte Bedeutung besitzen, r 0, 1 oder 2 ist, s 0 oder 1 bedeutet mit der Massgabe, das $(r+s) \leqslant 2$ ist, und wobei M eine direkte Bindung, $-(R^{14})C(R^{15})-$, -S-, -S-S-, -SO-, $-SO_2-$, $-CH_2SCH_2-$, -0-, $-CH_2OCH_2-$ oder $-NR^{16}$ - ist, wobei $R^{14}$ und $R^{15}$ unabhängig voneinander Wasserstoff, geradkettiges oder verzweigtes $C_1$-$C_{20}$. Alkyl, das gegebenenfalls durch 1 bis 3 Schwefelatome unterbrochen sein kann, oder $C_6$-$C_{10}$Aryl sein können, oder wobei $R^{14}$ und $R^{15}$ zusammen mit dem gemeinsamen C-Atom einen fünf- oder sechsgliedrigen Ring bilden, der gegebenenfalls mit ein oder zwei $C_1$-$C_8$Alkylgruppen substituiert sein kann, und wobei $R^{16}$ Wasserstoff, geradkettiges oder verzweigtes $C_1$-$C_{20}$Alkyl oder Phenyl ist; wobei die Verbindung der Formel I nureinen Rest der Formel III oder IV enthält, und wobei dieser Rest in 2- 4-oder 6-Position zur Hydroxygruppe der Formel I steht, und wobei im Fall, dass p= 1, $R^1 = C_1$-$C_{12}$ Alkyl, $R^2$ und $R^3 = C_1$-$C_5$Alkyl, k=1, $Q = -COOR^4$ und $R^4$ =wasserstoff sind, n nicht 2 ist;

zusätzlich betrifft die Erfindung Salze der Verbindungen der Formel I mit organischen oder anorganischen Säuren und Basen.

$R^1$ ist als geradkettiges oder verzweigtes $C_1$-$C_{12}$Alkyl beispielsweise Methyl, Ethyl, n-Propyl, Isopropyl, n-Butyl, sek.Butyl, tert.Butyl, tert.Amyl, 1,1,3,3-Tetramethylbutyl, n-Decyl oder n-Dodecyl.

$R^2$ und $R^3$ sind als geradkettiges oder verzweigtes $C_1$-$C_5$Alkyl beispielsweise Methyl, Ethyl, n-Propyl, Isopropyl, n-Butyl, sek.Butyl, n-Pentyl oder Neopentyl.

Als geradkettiges oder verzweigtes $C_1$-$C_{20}$Alkyl das gegebenenfalls von 1 bis 5 Sauerstoffatomen unterbrochen sein kann ist $R^4$ beispielsweise Methyl, Ethyl, 2-Methoxyethyl, 2-Ethoxyethyl, 2-n-Propoxyethyl, 2-n-Butoxyethyl, n-Pentyl, n-Hexyl, n-Heptyl, n-Octyl, 2-Ethylhexyl, n-Nonyl, n-Decyl, n-Dodecyl, n-Tetradecyl, n-Hexadecyl, n-Octadecyl, n-Eicosyl, $-(C_2H_4O)_2CH_3$, $-(C_2H_4O)_3CH_3$, $-(C_2H_4O)_4CH_3$ oder $-(C_2H_4O)_5CH_3$.

$R^4$, $R^6$ oder $R^7$ als geradkettige oder verzweigte $C_3$-$C_{20}$Alkenylgruppen sind beispielsweise Prop-2-enyl, n-But-2-enyl, 2-Methylprop-2-enyl, n-Pent-2-enyl, n-Hex-2-enyl, n-Hexa-2,4-dienyl, n-Dec-10-enyl oder n-Eicos-2-enyl.

Als $C_3$-$C_{12}$Cycloalkyl sind $R^4$, $R^6$ oder $R^7$ beispielsweise Cyclopropyl, Cyclobutyl, Cyclopentyl, Cyclohexyl, Cyclooctyl, Cyclodecyl, Adamantyl oder Cyclododecyl.

Als $C_7$-$C_{13}$Aralkyl sind $R^4$, $R^6$ oder $R^7$ beispielweise Benzyl, Phenylethyl, Benzhydryl oder Naphthylmethyl.

Als $C_6$-$C_{10}$Aryl sind R, $R^5$ oder $R^7$ beispielsweise Phenyl oderNaphthyl oder als ein- oder zweifach $C_1$-$C_4$ alkylsubstituiertes $C_6$-$C_{10}$Aryl sind $R^4$, $R^5$ oder $R^7$ beispielsweise Tolyl, Xylyl, Cumyl oder Butylphenyl.

Wenn Q -$COOR^4$ ist und wenn $R^4$ ein fünf- oder sechsgliedriger sauerstoffhaltiger gegebenenfalls mit einer oder zwei $C_1$-$C_4$Alkylgruppen substituierter Heterozyklus ist, kann es sich beispielsweise um Tetrafuran-3-yl, Tetrahydropyran-4-yl oder 2,6-Dimethyl-tetrahydro-pyran-4-yl handeln.

Wenn $R^4$ eine Methylgruppe ist, die einen sauerstoffhaltigen fünfoder sechsgliedrigen gegebenenfalls durch $C_1$-$C_4$Alkyl substituierten Heterozyklus trägt, so handelt es sich bei dem Ring beispielsweise um Furan, Tetrahydrofuran oder Tetrahydropyran.

$R^8$, $R^9$ und $R^{13}$ sind als geradkettiges oder verzweigtes $C_1$-$C_4$Alkyl beispielsweise Methyl, Ethyl, n-Propyl, Isopropyl, n-Butyl oder sek.Butyl.

Wenn $R^4$ und $R^5$ bzw. $R^8$ und $R^9$ zusammen mit dem Stickstoffatom, an das sie gebunden sind, einen fünf- oder sechsgliedrigen heterozyklischen Ring bilden, der gegebenenfalls durch eine oder zwei $C_1$-$C_4$Alkylgruppen substituiert sein kann, so ist dieser Ring beispielsweise Pyrrolidin, Piperidin, Morpholin oder 2,5-Dimethylmorpholin.

Wenn $R^5$ $R^7$ $R^{10}$ $R^{11}$ $R^{12}$ $R^{14}$ $R^{15}$ und $R^{16}$ unabhängig voneinander geradkettige oder verzweigte $C_1$-$C_{20}$Alkylgruppen sind, so handelt es sich beispielsweise um Methyl, Ethyl, n-Propyl, Isopropyl, n-Butyl, sek.Butyl, n-Pentyl, n-Hexyl, n-Heptyl, n-Octyl, 2-Ethylhexyl, n-Nonyl, n-Decyl, n-Dodecyl, n-Tetradecyl, n-Hexadecyl, n-Octadecyl oder n-Eicosyl.

Wenn $R^{10}$ und $R^{11}$ so miteinander verbunden sind, dass sie eine gegebenenfalls durch ein oder mehrere $C_1$-$C_{20}$Alkylgruppen substituierte $C_2$ oder $C_3$Methylenkette bilden, so kann es sich beispielsweise handeln um $-CH_2-CH_2-$, $-CH_2-CH_2-CH_2-$, $-CH_2-CH(CH_3)-$, $-CH_2-CH(C_2H_5)-$,
$-CH_2-CH(C_{20}H_{41})-$, $-CH(CH_3)-CH(CH_3)-$, $-CH(CH_3)-C(CH_3)_2-$, $-C(CH_3)_2C(CH_3)_2-$,
$-CH_2-CH_2-C(CH_3)_2-$ oder $-CH(CH_3)-CH_2-CH(CH_3)-$.

Wenn $R^{14}$ und $R^{15}$ zusammen mit dem Kohlenstoffatom an das sie gebunden sind einen gegebenenfalls substituierten $C_5$- oder $C_6$-Ring bilden, so handelt es sich beispielsweise um Cyclopentan, Cyclohexan, 4-tert.-Butylcyclohexan oder 2,6-Dimethylcyclohexan.

$R^{40}$ und $R^{41}$ sind als divalente geradkettige oder verzweigte $C_2$-$C_{20}$Alkylengruppen beispielsweise $-CH_2-CH_2-$, $-(CH_2)_3-$, $-(CH_2)_4$, $-(CH_2)_5-$,
$-(CH_2)_6-$, $-(CH_2)_7-$, $-(CH_2)_8-$, $-(CH_2)_{10}-$, $-(CH_2)_{12}-$, $-(CH_2)_{14}-$,
$-(CH_2)_{16}-$, $-(CH_2)_{18}-$, $-(CH_2)_{20}-$, $-CH_2-CH(CH_3)-$, $-CH_2-C(CH_3)_2-$,
$-CH(CH_3)-C(CH_3)-$ oder $-C(CH_3)_2-C(CH_3)_2-$.

Q und $R^1$ sind als Halogen, Fluor, Chlor, Brom oder Iod.

$R^{12}$ ist als Halogen Chlor oder Brom.

Salze von Verbindungeq der Formel I können mit Kationen der Alkalioder der Erdalkalimetalle oder mit Aminen gebildet werden. Im Fall von Q als $-NR^8$ $R^9$ entstehen Salze mit organischen und anorganischen Säuren wie beispielsweise Salzsäure, Schwefelsäure, p-Toluolsulfonsäure oder Oxalsäure.

Bevorzugt werden Verbindungen der Formel V

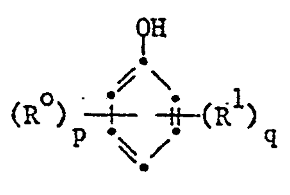

(V) ,

wobei $R^1$, p und q die gleiche Bedeutung wie oben definiert besitzen und wobei R° ein Rest der Formel IIa oder IIb ist

$$-\overset{R^2}{\underset{R^3}{C}}-C_nH_{2n+1-k}-(COOR^4)_k \quad (IIa), \qquad -\overset{R^2}{\underset{R^3}{C}}-C_nH_{2n+1-k}-(CONR^4R^5)_k \quad (IIb),$$

worin n, k, $R^2$, $R^3$, $R^4$ und $R^5$ die gleiche Bedeutung wie oben besitzen.

Besonders bevorzugt werden Substanzen der Formel V, worin k 1 ist.

Ebenfalls von Interesse sind Verbindungen der Formel V, die einen Rest der Formel IIa enthalten.

Zusätzlich bevorzugt werden Substanzen der Formel V, bei denen $R^2$ und $R^3$ Methyl bedeuten, und bei denen $R^1$ geradkettiges oder verzweigtes $C_1$-$C_5$Alkyl ist.

Besondere Beachtung finden Verbindungen der Formel V mit $n = 3$.

Ebenfalls bevorzugt sind Verbindungen der Formel I, bei denen $p = 1$ ist.

Besonders bevorzugt werden Verbindungen der Formel I mit $k = 1$.

Ebenfalls von Interesse sind Verbindungen der Formel I, bei denen $R^2$ und $R^3$ Methyl sind und $R^1$ geradkettiges oder verzweigtes $C_1$-$C_5$Alkyl ist.

Besondere Beachtung finden Verbindungen der Formel I mit n von 3 bis 10.

Insbesondere finden Verbindungen der Formel V Beachung, die weder einen Rest der Formel III noch einen Rest der Formel IV tragen.

Die folgende Beispiele für Verbindungen der Formel 1 erläutern die Erfindung ohne sie zu begrenzen:

5-(2-Hydroxyphenyl)-5-methyl-hexancarbonsäure und deren Natriumsalz
5-(4-Hydroxyphenyl)-5-methyl-hexancarbonsäure und deren Natriumsalz
Methyl-5-(2-hydroxyphenyl)-5-methyl-hexanoat
Methyl-5-(4-hydroxyphenyl)-5-methyl-hexanoat
n-Hexyl-5-(4-hydroxyphenyl)-5-methyl-hexanoat
2-Ethylhexyl-5-(4-hydroxyphenyl)-5-methyl-hexanoat
Eicosyl-5-(4-hydroxyphenyl)-5-methyl-hexanoat
2-Butoxyethyl-5-(4-hydroxyphenyl)-5-methyl-hexanoat
2-Cyclohexyloxyethyl-5-(4-hydroxyphenyl)-5-methyl-hexanoat
2-Allyloxyethyl-5-(4-hydroxyphenyl)-5-methyl-hexanoat
2-phenoxyethyl-5-(4-hydroxyphenyl)-5-methyl-hexanoat
2-(4-Methylphenoxy)-ethyl-5-(4-hydroxyphenyl)-5-methyl-hexanoat
2-Benzyloxyethyl-5-(4-hydroxyphenyl)-5-methyl-hexanoat
Allyl-5-(4-hydroxyphenyl)-5-methyl-hexanoat
Undecyl-5-(4-hydroxyphenyl)-5-methyl-hexanoat
Cyclohexyl-5-(4-hydroxyphenyl)-5-methyl-hexanoat
Phenyl-5-(4-hydroxyphenyl)-5-methyl-hexanoat
4-Methylphenyl-5-(4-hydroxyphenyl)-5-methyl-hexanoat
Benzyl-5-(4-hydroxyphenyl)-5-methyl-hexanoat
Tetrahydrofurfuryl-5-(4-hydroxyphenyl)-5-methyl-hexanoat
Furfuryl-5-(4-hydroxyphenyl)-5-methyl-hexanoat
Tetrahydropyran-4-yl-5-(4-hydroxyphenyl)-5-methyl-hexanoat
5-(4-Hydroxyphenyl)-5-methyl-hexansäureamid
N-Methyl-5-(4-hydroxyphenyl)-5-methyl-hexansäureamid
N-n-Butyl-5-(4-hydroxyphenyl)-5-methyl-hexansäureamid
N,N-Dimethyl-5-(4-hydroxyphenyl)-5-methyl- hexansäureamid
N,N-Diethyl-5-(4-hydroxyphenyl)-5-methyl-hexansäureamid
N,N-Di-n-butyl-5-(4-hydroxyphenyl)-5-methyl-hexansäureamid
N-n-Eicosyl-5-(4-hydroxyphenyl)-5-methyl-hexansäureamid
N-Allyl-5-(4-hydroxyphenyl)-5-methyl-hexansäureamid
N-Cyclohexyl-5-(4-hydroxyphenyl)-5-methyl-hexansäureamid
N-Benzyl-5-(4-hydroxyphenyl)-5-methyl- hexansäuresmid
N-Phenyl-5-(4-hydroxyphenyl)-5-methyl-hexansäureamid
N-[5-(4-Hydroxyphenyl)-5-methyl-hexanoyl]-morpholin
Methyl-5-(4-hydroxy-3-methylphenyl)-5-methyl-hexanoat
Allyl-5-(4-hydroxy-3-methylphenyl)-5-methyl-hexanoat
Cyclohexyl-5-(4-hydroxy-3-methylphenyl)-5-methyl-hexanoat
Methyl-5-(2-hydroxy-5-methylphenyl)-5-methyl-hexanoat
5-(2-Hydroxy-5-methylphenyl)-5-methyl-hexancarbonsäure
5-(2-Hydroxy-3,5-di-methylphenyl)-5-methyl-hexancarbonsäure
Methyl-5-(2-hydroxy-3,5-di-methylphenyl)-5-methyl-hexanoat
Methyl-5-(4-hydroxy-3,5-di-methylphenyl)-5-methyl-hexanoat
5-(5-Ethyl-2-hydroxyphenyl)-5-methyl-hexancarbonsäure

Methyl-5-(5-ethyl-2-hydroxyphenyl)-5-methyl-hexanoat
Methyl-5-(3-ethyl-4-hydroxyphenyl)-5-methyl-hexanoat
Methyl-5-(3,5-di-ethyl-4-hydroxyphenyl)-5-methyl-hexanoat
Methyl-5-(5-n-decyl-2-hydroxyphenyl)-5-methyl-hexanoat
5-(2-Hydroxy-5-isopropylphenyl)-5-methyl-hexancarbonsäure
Methyl-5-(2-hydroxy-5-isopropylphenyl)-5-methyl-hexanoat
Methyl-5-(4-hydroxy-3-isopropylphenyl)-5-methyl-hexanoat
Methyl-5-(2-hydroxy-3,5-di-isopropylphenyl)-5-methyl-hexanoat
Methyl-5-(4-hydroxy-3,5-di-isopropylphenyl)-5-methyl-hexanoat
Methyl-5-(3-sec-butyl-4-hydroxyphenyl)-5-methyl-hexanoat
Methyl-5-(3-t-butyl-2-hydroxy-5-methylphenyl)-5-methyl-hexanoat
Methyl-5-(3-t-butyl-4-hydroxy-5-methylphenyl)-5-methyl-hexanoat
Methyl-5-(3-cumyl-4-hydroxyphenyl)-5-methyl-hexanoat
Methyl-5-(3-cumyl-4-hydroxy-5-methylphenyl)-5-methyl-hexanoat
Methyl-5-(3-t-butyl-2-hydroxy-5-isopropylphenyl)-5-methyl-hexanoat
Methyl-5-(3,5-di-t-butyl-2-hydroxyphenyl)-5-methyl-hexanoat
Methyl-5-(3,5-di-t-butyl-4-hydroxyphenyl)-5-methyl-hexanoat
Methyl-5-[2-hydroxy-5-(1,1,3,3-tetramethylbutyl)-phenyl]-5-methyl-hexanoat
5-(3-tert.Butyl-2-hydroxy-5-methylphenyl)-5-methyl-hexansäure
5-(3-tert.Butyl-2-hydroxy-5-methylphenyl)-5-methyl-hexansäure-chlorid
Bis-[5-(3-tert.butyl-4-hydroxy-5-methylphenyl)-5-methyl -hexansäure]-ester von 1,6-Hexandiol
Bis-[5-(3-t.butyl-4-hydroxy-5-isopropylphenyl)-5-methyl -hexansäure]-ester von 1,6-Hexandiol
Bis-[5-(3,5-di-tert.butyl-4-hydroxyphenyl)-5-methyl-hexansäure]-ester von 1,6-Hexandiol
5-(3-tert.Butyl-2-hydroxy-5-methylphenyl)-5-methyl-hexansäure-amid von 1,1,3,3-Tetramethylbutylamin
5-(3-tert.Butyl-2-hydroxy-5-methylphenyl)-5-methyl-hexansäure-amid von n-Dodecylamin
Bis-5-(3-tert.butyl-2-hydroxy-5-methylphenyl)-5-methyl-hexansäureamid von 1,6-Diamino-hexan
Methyl-5-(5-chloro-2-hydroxyphenyl)-5-methyl-hexanoat
Methyl-5-(3-chloro-2-hydroxyphenyl)-5-methyl-hexanoat
Methyl-5-(3-chloro-4-hydroxyphenyl)-5-methyl-hexanoat
Methyl-5-(3,5-dichloro-4-hydroxyphenyl)-5-methyl-hexanoat
2,4-Bis-(5-methoxycarbonyl-2-methyl-pent-2-yl)-phenol
2,6-Bis-(5-methoxycarbonyl-2-methyl-pent-2-yl)-phenol
2,6-Bis-(5-methoxycarbonyl-2-methyl-pent-2-yl)-4-methyl-phenol
2,4,6-Tris-(5-carboxy-2-methyl-pent-2-yl)-phenol
2,4,6-Tris-(5-methoxycarbonyl-2-methyl-pent-2-yl)-phenol
2,4-Bis-(5-carboxy-2-methyl-pent-2-yl)-phenol
2,6-Bis-(5-carboxy-2-methyl-pent-2-yl)-4-methyl-phenol
2-(8-n-(Butyloxy-2,6-dimethyl-oct-2-yl)-4-(5-methoxycarbonyl-2-methyl-pent-2-yl)-phenol
2,6-Bis-(5-carboxy-2-methyl-pent-2-yl)-phenol
4-(7-Methoxycarbonyl-2,2,4-trimethyl-hept-4-yl)-phenol
Methyl-5-(4-hydroxyphenyl)-2-methoxycarbonyl-5-methyl-hexanoat
2-Carboxy-5-(2-hydroxy-5-methylphenyl)-5-methyl-hexansäure
Methyl-5-(2-hydroxy-5-methylphenyl)-2-methoxycarbonyl-5-methyl-hexanoat
Dimethyl-5-methyl-5-(2-hydroxyphenyl)-azelat
Dimethyl-5-methyl-5-(4-hydroxyphenyl)-azelat
7-(4-Hydroxyphenyl)-3,7-dimethyl-octan-1-ol
7-(2-Hydroxy-5-methylphenyl)-3,7-dimethyl-octan-1-ol
1-n-Butyloxy-7-(4-hydroxy-3,5-dimethylphenyl)-3,7-dimethyloctan
1-Acetoxy-7-(4-hydroxyphenyl)-3,7-dimethyl-octan
1-Eicosoyloxy-7-(4-hydroxyphenyl)-3,7-dimethyl-octan
1-Crotonyloxy-7-(4-hydroxyphenyl)-3,7-dimethyl-octan
1-Cyclohexancarbonyloxy-7-(4-hydroxyphenyl)-3,7-dimethyl-octan
1-Phenylacetoxy-7-(4-hydroxyphenyl)-3,7-dimethyl-octan
1-Benzoyloxy-7-(4-hydroxyphenyl)-3,7-dimethyl-octan
1-(4-Methylbenzoyloxy)-7-(4-hydroxyphenyl)-3,7-dimethyl-octan
6-(2-Hydroxyphenyl)-6-methyl-2-methylamino-heptan
6-(2-Hydroxyphenyl)-6-methyl-2-dimethylamino-heptan
2-n-Ethylamino-6-(4-hydroxyphenyl)-6-methyl-heptan
2-n-Butylamino-6-(2-hydroxyphenyl)-6-methyl-heptan
2-di-n-Butylamino-6-(2-hydroxyphenyl)-6-methyl-heptan
6-(2-Hydroxyphenyl)-6-methyl-2-morpholino-heptan
2-Amino-6-(2-hydroxyphenyl)-6-methyl-heptan und sein Hydrochlorid
2-Amino-6-(4-hydroxyphenyl)-6-methyl-heptan und sein Hydrochlorid
2-Acetamido-6-(2-hydroxyphenyl)-6-methyl-heptan
2-Acetamido-6-(4-hydroxyphenyl)-6-methyl-heptan

2-Eicosamido-6-(4-hydroxyphenyl)-6-methyl-heptan
2-Crotonamido-6-(4-hydroxyphenyl)-6-methyl-heptan
2-Cyclohexancarbonamido-6-(4-hydroxyphenyl)-6-methyl-heptan
2-Benzamido-6-(4-hydroxyphenyl)-6-methyl-heptan
Dimethyl-3-(2-hydroxyphenyl)-3-methyl-butan-phosphonat
Dimethyl-3-(4-hydroxyphenyl)-3-methyl-butan-phosphonat
Diethyl-3-(4-hydroxyphenyl)-3-methyl-butan-phosphonat
Dimethyl-3-(2-hydroxy-5-methylphenyl)-3-methyl-butan-phosphonat
Dimethyl-3-(4-hydroxy-3,5-dimethylphenyl)-3-methyl-butan-phosphonat
Dimethyl-3-(4-hydroxy-3,5-di-isopropylphenyl)-3-methyl-butan-phosphonat
Diethyl-7-(4-hydroxyphenyl)-3,7-dimethyl-octan-phosphonat
Diethyl-2-ethoxycarbonyl-5-(2-hydroxyphenyl)-5-methyl-hexan-phosphonat
Diethyl-2-ethoxycarbonyl-5-(4-hydroxyphenyl)-5-methyl-hexan-phosphonat
3-(2-Hydroxyphenyl)-3-methyl-butan-phosphonsäure
3-(2-Hydroxy-5-methylphenyl)-3-methyl-butan-phosphonsäure
3-(2-Hydroxyphenyl)-3-methyl-butan-methyl-phosphonsäure und deren Natriumsalz
Di-dodecyl-3-(4-hydroxyphenyl)-3-methyl-butyl-phosphonat
2-[3-(4-Hydroxyphenyl)-3-methyl-butyl]- 2-oxo-1,3,2-dioxa-phospholan
1-Bromo-7-(4-hydroxyphenyl)-3,7-dimethyl-octan
7-(2-Hydroxyphenyl)-3,7-dimethyl-1-nitro-octan
7-(4-Hydroxyphenyl)-3,7-dimethyl-1-nitro-octan
1-Acetoxy-3-(4-hydroxyphenyl)-3-methyl-butan
6-(2-Hydroxyphenyl)-6-methyl-heptan-2-on
6-(4-Hydroxyphenyl)-6-methyl-heptan-2-on
Cis-[4-acetyl-1-(4-hydroxyphenyl)]-1-methyl-cyclohexan
Trans-[4-acetyl-1-(4-hydroxyphenyl)]-1-methyl-cyclohexan
1-Cyano-4-(2-hydroxyphenyl)-4-methyl-pentan
1-Cyano-4-(4-hydroxyphenyl)-4-methyl-pentan
Cis-[4-cyano-1-(4-hydroxyphenyl)]-1-methyl-cyclohexan
Trans-[4-cyano-1-(4-hydroxyphenyl)]-1-methyl-cyclohexan
Ethyl-2-cyano-5-(4-hydroxyphenyl)-5-methyl-hexanoat
Cis-[1-(4-hydroxyphenyl)-4-methoxycarbonyl]-1-methyl-cyclohexan
Trans-[1-(4-hydroxyphenyl)-4-methoxycarbonyl]-1-methyl-cyclohexan
Cis-[1-(2-hydroxyphenyl)-4-methoxycarbonyl]-1-methyl-cyclohexan
Trans-[1-(2-hydroxyphenyl)-4-methoxycarbonyl]-1-methyl-cyclohexan
Cis-[1-(4-Hydroxyphenyl)-cis-3,4-di-methoxycarbonyl]-1-methyl-cyclohexan
Trans-[1-(4-hydroxyphenyl)-cis-3,4-di-methoxycarbonyl]-1-methyl-cyclohexan
Cis-[1-(2-hydroxy-5-methylphenyl)-4-methoxycarbonyl]-1-methyl-cyclohexan
Trans-[1-(2-hydroxy-5-methylphenyl)-4-methoxycarbonyl]-1-methyl-cyclohexan
Cis-[1-(4-hydroxy-3,5-di-isopropylphenyl)-4-methoxycarbonyl]-1-methyl-cyclohexan
Trans-[1-(4-hydroxy-3,5-di-isopropylphenyl)-4-methoxycarbonyl]-1-methyl-cyclohexan
Trans-[1-(4-hydroxy-3,5-di-isopropylphenyl)-4-carboxy]-1-methyl-cyclohexan
Trans-[1-(3,5-di-t-butyl-4-hydroxyphenyl)-4-methoxycarbonyl]-1-methyl-cyclohexan
Bis-[4-methyl-trans-(4-(3,5-di-t-butyl-4-hydroxyphenyl)-cyclohexane-1-carbonsäure)]-ester von Hexan-1,6-diol
5-(5-Carboxy-2-methyl-pent-2-yl)-2-hydroxy-benzoesäure
Methyl-2-hydroxy-5-(5-methoxycarbonyl-2-methyl-pent-2-yl)-benzoat
3,5-Bis-(5-carboxy-2-methyl-pent-2-yl)-2-hydroxy-benzoesäure
Methyl-2-hydroxy-3,5-bis-(5-methoxycarbonyl-2-methyl-pent-2-yl)-benzoat
5-(6-Amino-2-methyl-hept-2-yl)-2-hydroxy-benzoesäure
Methyl-5-(6-amino-2-methyl-hept-2-yl)-2-hydroxy-benzoat
2-Hydroxy-5-(2-methyl-4-phosphon-but-2-yl)-benzoesäure
Methyl-2-hydroxy-5-(2-methyl-4-dimethylphosphon-but-2-yl)-benzoat
Methyl-4-hydroxy-3-(5-methoxycarbonyl-2-methyl-pent-2-yl)-benzoat
2-Hydroxy-5-(5-methoxycarbonyl-2-methyl-pent-2-yl)-acetophenon
2-Hydroxy-3-(5-methoxycarbonyl-2-methyl-pent-2-yl)-acetophenon
4-Hydroxy-3-(5-methoxycarbonyl-2-methyl-pent-2-yl)-acetophenon
2-Hydroxy-5-(5-methoxycarbonyl-2-methyl-pent-2-yl)-benzophenon
2-Hydroxy-3-(5-methoxycarbonyl-2-methyl-pent-2-yl)-benzophenon
4-Hydroxy-3-(5-methoxycarbonyl-2-methyl-pent-2-yl)-benzophenon
2-Cyano-4-(5-methoxycarbonyl-2-methyl-pent-2-yl)-phenol
4-Cyano-2-(5-methoxycarbonyl-2-methyl-pent-2-yl)-phenol
2-Hydroxy-5-(5-methoxycarbonyl-2-methyl-pent-2-yl)-benzamid
4-Hydroxy-3-(5-methoxycarbonyl-2-methyl-pent-2-yl)-benzamid
4-(5-Methoxycarbonyl-2-methyl-pent-2-yl)-2-methylmercapto-phenol
2-(5-Methoxycarbonyl-2-methyl-pent-2-yl)-4-methylmercapto-phenol

4-(5-Methoxycarbonyl-2-methyl-pent-2-yl)-2-trifluormethyl-phenol
2-Hydroxy-5-(5-methoxycarbonyl-2-methyl-pent-2-yl)-benzol-sulfonsäure
2-Hydroxy-5-(5-methoxycarbonyl-2-methyl-pent-2-yl)-benzol-sulfonamid
2-Hydroxy-5-(5-methoxycarbonyl-2-methyl-pent-2-yl)-benzol-phosphon-saure
Diethyl-2-hydroxy-5-(5-methoxycarbonyl-2-methyl-pent-2-yl)-phenyl-phosphonat
4-(5-Methoxycarbonyl-2-methyl-pent-2-yl)-2-nitrophenol
Bis-[2-hydroxy-5-(5-methoxycarbonyl-2-methyl-pent-2-yl)-3-methyl-phenyl]-methan
Bis-[2-hydroxy-3-(5-methoxycarbonyl-2-methyl-pent-2-yl)-5-methyl-phenyl]-methan
Bis-[4-hydroxy-5-(5-methoxycarbonyl-2-methyl-pent-2-yl)-3-methyl-phenyl]-methan
Bis-2,2-[4-hydroxy-5-(5-methoxycarbonyl-2-methyl-pent-2-yl)-3-methyl-phenyl]-propan
Bis-1,1-[2-hydroxy-5-(5-methoxycarbonyl-2-methyl-pent-2-yl)-3-methyl-phenyl]-cyclohexan
Bis-[2-hydroxy-5-(5-methoxycarbonyl-2-methyl-pent-2-yl)-3-methyl-phenyl]-sulfid
Bis-[2-hydroxy-5-(5-methoxycarbonyl-2-methyl-pent-2-yl)-3-methyl-phenyl]-disulfid
Bis-[2-hydroxy-3-(5-methoxycarbonyl-2-methyl-pent-2-yl)-5-methyl-phenyl]-sulfoxid
Bis-[2-hydroxy-5-(5-methoxycarbonyl-2-methyl-pent-2-yl)-3-methyl-phenyl]-sulfon
Bis-[2-hydroxy-3-(5-methoxycarbonyl-2-methyl-pent-2-yl)-3-methyl-benzyl]-sulfid
Bis-[4-hydroxy-5-(5-methoxycarbonyl-2-methyl-pent-2-yl)-3-methyl-phenyl]-oxid
Bis-[2-hydroxy-5-(5-methoxycarbonyl-2-methyl-pent-2-yl)-3-methyl-benzyl]-oxid
4,4-Dihydroxy-5,5'-(5-methoxycarbonyl-2-methyl-pent-2-yl)-3,3'-dimethyl-diphenylamin.

Die vorliegende Erfindung betrifft ein Verfahren zur Herstellung von Verbindungen der Formel I, dadurch gekennzeichnet, dass ein Phenol der Formel VI in Gegenwart eines geeigneten Katalysators mit einem Alkylierungsreagenz unter Einführung einer Gruppe der Formel II umgesetzt wird

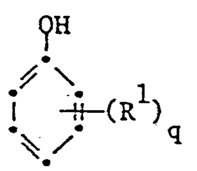 (VI) ,

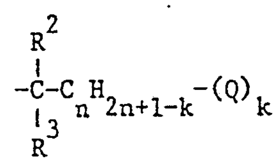 (II) ,

worin $R^1$, $R^2$, $R^3$, Q, n, q und k die oben definierte Bedeutung besitzen, und worin die Verbindung VI nur einen der Reste III oder IV in 2-, 4- oder 6-Position zur Hydroxygruppe trägt.

Die Alkylierung wird im Temperaturbereich zwischen 20°C und 170°C durchgeführt, bevorzugt aber zwischen 100°C und 150°C. Als Katalysator kann eine Brönstedsäure, eine saure Erde oder ein Metallsalz verwendet werden. Geeignete Brönstedsäuren können organischer oder anorganischer Natur sein oder es kann sich um saure Salze oder um Mineralsäuren wie Salzsäure, Schwefelsäure, Perchlorsäure oder Orthophosphorsäure handeln, oder es können alkyl-, aryl- oder aralkylsubstituierte anorganische Säuren wie beispielsweise Methan- oder Ethansulfonsäuren, Benzolsulfonsäure, p-Toluolsulfonsäure oder Methylphosphonsäure sein.

Als organische Säuren können beispielsweise Dichloressigsäure, Trichloressigsäure oder Trifluoressigsäure Verwendung finden.

Bei sauren Erden handelt es sich beispielsweise um Fulmont 237® oder Fulcat 22®, während Aluminiumphenoxid ein geeignetes Metallsalz darstellt.

Bevorzugt als Katalysatoren werden saure Erden.

Die Reaktion kann mit oder ohne Lösungsmittel ausgeführt werden. Gängige Lösungsmittel sind Kohlenwasserstoffe wie Benzol, Cyclohexan oder Heptan. Daneben können Methanol/Schwefelsäure oder Wasser/Schwefelsäure verwendet werden, wobei die Säure hier gleichzeitig sls Katalysator dient.

Verbindungen der Formel I erhält man durch Umsetzung von einem Mol des Phenols der Formel VI mit 0,1 bis 4,0 Molen eines Alkylierungsmittels.

Wenn Substanzen der Formel I mit $p=1$ und $q=0$, 1 oder 2 hergestellt werden sollen, verwendet man 0,1 bis 1,0 Mole des Alkylierungsmittels pro Mol der Ausgangsverbindung VI.

Im Fall von Substanzen der Formel I mit $p=2$ und $q=0$ oder 1 verwendet man wenigstens 2 Mole des Alkylierungsmittels pro Mol der Ausgangsverbindung VI.

Wenn Substanzen der Formel I mit $p=3$ und $q=0$ hergestellt werden sollen, verwendet man wenigstens 3 Mole des Alkylierungsmittels pro Mol der Ausgangsverbindung VI.

Wenn $R^1$ ein Rest der Formel III oder IV ist, bei dem s und/oder r 0 oder 1 sind, verwendet man bis zu 4 Molen des Alkylierungsmittels pro Mol der Ausgangsverbindung VI.

Gegebenenfalls kann man Verbindungen der Formel I, bei denen p 2 oder 3 ist und bei denen die Gruppen der Formel II gleich oder unterschiedlich sind, herstellen, indem man die Gruppe II stufenweise in die Verbindung der Formel I einführt, indem man jeweils ein Mol des Alkylierungsmittels pro Mol der Verbindung I einsetzt.

Auf die gleiche Weise können Verbindungen der Formel I hergestellt werden, bei denen p 1 oder 2 ist, q 1 oder 2 ist und $R^1$ $C_4$-$C_8$Alkyl ist, indem man von Verbindungen der Formel 1 mit $q=0$ oder 1 und $p = 1$ oder 2 ausgeht und diese durch Alkylierung mit wenigsten einem Mol eines $C_4$-$C_8$Olefins oder $C_4$-$C_8$Alkohols in Gegenwart eines sauren Katalysators umsetzt.

Substanzen der Formel I, die einen Rest III oder IV enthalten, können nach herkömmlichen Methoden erhalten werden, indem man 2 Mole der Verbindung I, in der q=0 und p=1 oder 2 bedeuten, mit einem Reagenz vernetzt, welches zur Bildung der Brücke M führt.

Besonders bevorzugt wird ein Verfahren zur Herstellung von Verbindungen der Formel I, worin $R^1$ ein Rest der Formel III oder VI ist, und worin s und/oder r 0 oder 1 sind, dadurch gekennzeichnet, dass man bis zu 4 Mol eines Alkylierungsmittels pro Mol des Ausgangsphenols VI verwendet.

Phenole der Formel VI sind beispielsweise:

Phenol
o-Kresol
m-Kresol
p-Kresol
2,6-Xylenol
2,4-Xylenol
2-Ethylphenol
4-Ethylphenol
2,6-Diethylphenol
2-Isopropylphenol
4-Isopropylphenol
2,6-Di-isopropylphenol
2-sek.Butylphenol
4-sek.Butylphenol
2,6-Di-sek.butylphenol
4-tert.Butylphenol
4-(1,1,3,3-Tetramethylbutyl)-phenol
4-n-Decylphenol
2-Chlorphenol
4-Chlorphenol
2,6-Dichlorphenol
2-Chlor-4-methylphenol
Salicylsäure
Methylsalicylat
4-Hydroxybenzoesäure
Methyl-p-hydroxy-benzoat
2-Hydroxyacetophenon
2-Hydroxybutyrophenon
4-Hydroxyacetophenon
2-Hydroxybenzophenon
4-Hydroxybenzophenon
2-Cyanophenol
4-Cyanophenol
2-Hydroxybenzamid
4-Hydroxybenzamid
2-Methylmercaptophenol
4-Methylmercaptophenol
4-n-Butylmercaptophenol
2-Trifluormethylphenol
4-Trifluormethylphenol
2-Hydroxybenzolsulfonsäure
4-Hydroxybenzolsulfonsäure
2-Hydroxybenzolsulfonamid
4-Hydroxybenzolsulfonamid
2-Hydroxybenzolphosphonsäure
4-Hydroxybenzolphosphonsäure
Diethyl-2-hydroxyphenylphosphonat
Diethyl-4-hydroxyphenylphosphonat
2-Nitrophenol
4-Nitrophenol
4,4'-Dihydroxydiphenylmethan
4,4'-Dihydroxy-3,3'-dimethyl-diphenylmethan
2,2'-(4,4'-Dihydroxydiphenyl)-propan
1,1'-(4,4'-Dihydroxydiphenyl)-cyclohexan
4,4'-Dihydroxydiphenylamin
4,4'-Dihydroxydiphenylether.

Alkylierungsreagenzien, die mit dem Phenol VI umgesetzt werden, enthalten eine reaktive Gruppe, beispielsweise einen olefinischen Rest oder eine Hydroxygruppe, die während der Alkylierung eliminiert,

umgeformt oder ausgetauscht wird.

Funktionelle Olefine, die zur Alkylierung von Verbindungen der Formel VI eingesetzt werden können, sind beispielsweise:

5-Methyl-hex-5-ensäure
Methyl-5-methyl-hex-4-enoat
Methyl-5-methyl-hex-5-enoat
Ethyl-5-methyl-hex-5-enoat
n-Hexyl-5-methyl-hex-5-enoat
2-Ethylhexyl-5-methyl-hex-5-enoat
n-Hexadecyl-5-methyl-hex-5-enoat
Methyl-5,7,7-trimethyl-oct-4-enoat
1,7-Dimethoxycarbonyl-4-methyl-hept-3-en
1-Acetoxy-3-methyl-but-3-en
Citronellol
Citronellylacetat
Citronellyl-n-butylether
Citronellylcarbonsäure
Methylcitronellat
Citronellylnitril
1-Bromo-3,7-dimethyl-oct-6-en
3,7-Dimethyl-1-nitro-oct-6-en
Diethyl-3,7-dimethyl-oct-6-en-1-phosphonat
Methyl-2-methoxycarbonyl-5-methyl-hex-4-enoat
Ethyl-2-ethoxycarbonyl-5-methyl-hex-4-enoat
Diethyl-2-ethoxycarbonyl-5-methyl-hex-4-en-2-phosphonat
Ethyl-2-cyano-5-methyl-hex-4-enoat
Dimethyl-prenyl-phosphonat
Diethyl-prenyl-phosphonat
1-Cyano-4-methyl-pent-4-en
6-Methyl-hept-6-en-2-on
2-Amino-6-methyl-hept-5-en und Hydrochlorid
2-Amino-6-methyl-hept-6-en und Hydrochlorid
2-Acetamido-6-methyl-hept-5-en
2-Acetamido-6-methyl-hept-6-en
4-Methoxycarbonyl-1-methyl-cyclohex-1-en
4-Cyano-1-methyl-cyclohex-1-en
4-Acetyl-1-methyl-cyclohex-1-en.

Geeignete Hydroxyverbindungen für die Alkylierung von Substanzen der Formel VI sind beispielsweise:

2-Amino-6-hydroxy-6-methyl-heptan und Hydrochlorid
2-Acetamido-2-hydroxy-6-methyl-heptan
11-Amino-2,2,12-trimethyl-tridecan-1-ol,

und ebenso Verbindungen, die sich von 11-Aminoundecanolen der folgenden Formel VIII ableiten:

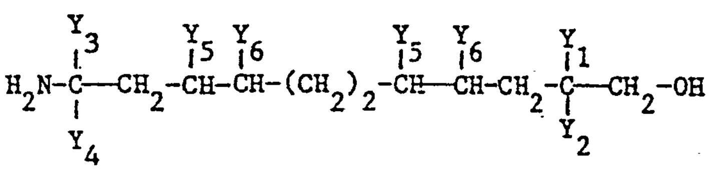

(VIII),

wobei $Y_1$ und $Y_3$ unabhängig voneinander Wasserstoff oder $C_1$-$C_8$Alkyl bedeuten, $Y_2$ und $Y_4$ unabhängig voneinander $C_1$-$C_8$Alkyl sind, und $Y_5$ und $Y_6$ unabhängig voneinander Wasserstoff oder $C_1$-$C_4$Alkyl sind.

Herstellungsweise und Eigenschaften dieser 11-Aminoundecanole sind umfassender in der DE-OS 2,831,299 beschrieben.

Für die Alkylierung von Phenolen der Formel I wobei q 0 oder 1 ist und p 1 oder 2 bedeutet, verwendet man beispielsweise Olefine wie Isobutylen oder Di-isobutylen, oder Alkohole wie tert.Butanol oder 1,1,3,3-Tetramethylbutan-1-ol.

Jedes beliebige funktionelle Derivat von Verbindungen der Formel I kann in ein anderes funktionelles Derivat umgewandelt werden. Wenn beispielsweise Q eine Carbonsäuregruppe -COOH ist, so kann man daraus durch Umsetzung mit dem einwertigen Alkohol $R^4OH$ einen Ester $-COOR^4$, oder durch Umsetzung mit dem zweiwertigen Alkohol $R^{40}(OH)_2$einen Ester $(-COO)_2R^{40}$ herstellen, oder man kann die Estergruppierung $-COOR^4$ umestern, und einen weiteren Ester mit einer neuen $R^4$ oder $R^{40}$-Gruppe erhalten, oder man kann den Ester $-COOR^4$ in ein Carbonsäureamid $-CONR^4R^5$ überführen, indem man ihn mit einem Amin $NHR^4R^5$ umsetzt, wobei $R^4$ und $R^5$ die oben definierte Bedeutung haben, oder indem man den Ester mit einem Dismin $R^5HN$-$R^{41}$-$NHR^5$ umsetzt, wobei die Reste $R^5$ unabhängig voneinander sein können.

Die Verbindungen I können als Stabilisatoren und/oder Antioxydantien für Polymere, Oele oder photographische Systeme verwendet werden, sie sind ebenfalls Korrosionsinhibitoren. Daneben stellen Verbindungen der Formel I nützliche Zwischenprodukte für Photochemikalien dar.

Die vorliegende Erfindung betrifft daher auch Zusammensetzungen enthaltend ein Substrat und als Stabilisator wenigsten eine Verbindung der Formel I.

In einer bevorzugten Ausführungsform liegt ein Zweiphasensystem vor bestehend aus einem wässrigen Medium in Kontakt zu Metallen, bevorzugt zu Eisenmetallen, welches in der wässrigen Phase einen Korrosionsinhibitor der Formel I enthält.

In einer weiteren bevorzugten Ausführungsform besteht das Substrat aus einem oxidationsempfindlichen organischen Material, beispielsweise einem organischen Polymeren, einem Mineralöl oder einem photographischen System, und aus einem Antioxydans der Formel I, bevorzugt verwendet als thermisches Antioxydans.

Die folgenden Beispiele erläutern die Erfindung näher ohne sie zu begrenzen. Teile und Prozentangaben beziehen sich auf das Gewicht, Drucke sind in Millibar angegeben.

**Beispiel 1:**

a) 94 Teile Phenol, 14,2 Teile Methyl-5-methyl-hex-5-enoat und 5,0 Teile Fulmont 237® werden bei 110°C 20 Stunden lang gerührt. Die Reaktionsmischung wird kurz abgekühlt, filtriert und anschliessend destilliert. Man erhält 82 Teile des Phenols zurück und gewinnt als Reaktionsprodukt Methyl-5-(4-hydroxyphenyl)-5-methylhexanoat vom $K.P._{0,65}$ 167-172°C.

Analyse: C H
berechnet 71,16 8,53
gefunden 71,01 8,60

b) 5,0 Teile Methyl-5-(4-hydroxyphenyl)-5-methylhexanoat, 5,0 Teile Natriumhydroxid und 50 Teile Wasser werden 3 Stunden unter Rückfluss gekocht. Aus der homogenen Lösung erhält man nach Ansäuern mit 36%iger Salzsäure ein Oel, das beim Abkühlen erstarrt. Der feste Rückstand wird abfiltriert, bei 0°C aus Petrolether (K.P. 40-60°C) umkristallisiert und ergibt 5-(4-Hydroxyphenyl)-5-methylhexansäure vom F.P. 94-96°C.

Analyse: C H
berechnet 70,24 8,16
gefunden 70,33 8,22

**Beispiel 2:** 94 Teile Phenol, 28,5 Teile

4-Methoxycarbonyl-1-methyl-cyclohexyl-1-en und 5,0 Teile Fulmont 237® werden wie in Beispiel 1 beschrieben umgesetzt und aufgearbeitet. Die Fraktion mit dem $K.P._{0,4}$ 167-194°C besteht aus cis- und trans-Isomeren von 1-(4-Hydroxyphenyl)-4-methoxycarbonyl-1-methylcyclohexan und wird fraktioniert aus Ether umkristallisiert. Das auf diese Weise erhaltene Rohprodukt wird aus Methanol/Wasser umkristallisiert und man erhält das trans-Isomere vom F.P. 120-122°.

Analyse: C H
berechnet 72,55 8,12
gefunden 72,01 7,91

Ausdenvereinigten Mutterlaugender fraktionierten Kristallisation wird eine zweite Fraktion gewonnen, die aus Methanol/Wasser umkristallisiert wird. Man erhält dar aus das cis-Isomere mit dem F.P. 128°C bis 130°C.

Analyse: C H
berechnet 72,55 8,12
gefunden 72,01 7,91

**Beispiele 3 bis 20:**

In der folgenden Tabelle 1a sind Verbindungen der Formel I aufgelistet, die in Analogie zu der Vorschrift in Beispiel 1 hergestellt wurden. Tabelle 1b enthält Analysedaten, Schmelz- oder Siedepunkte der einzelnen Verbindungen sowie Einzelheiten zur Reaktionsdurchführung.

**Tabelle 1a:**

| Beispiel | Phenol (Teile) | Alkylierungsmittel (Teile) | Produkte |
|---|---|---|---|
| 3 | 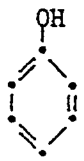 (94) | 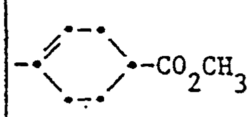 (30.8) | 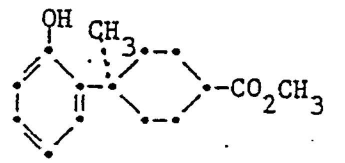 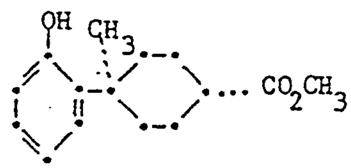 |
| 4 | 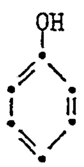 (94) | 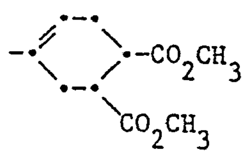 (21.2) | 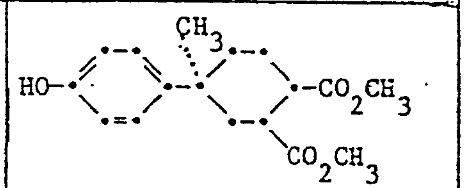 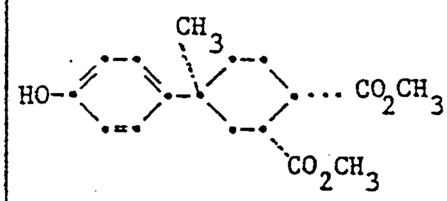 |

**Tabelle 1a:** (Fortsetzung)

| Beispiel | Phenol (Teile) | Alkylierungsmittel (Teile) | Produkte |
|---|---|---|---|
| 5 | 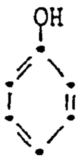 (94) | 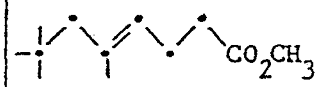 (39.6) | 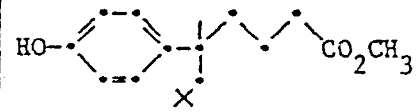 |
| 6 | 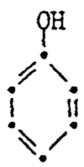 (14.1) | 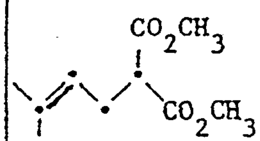 (84) | 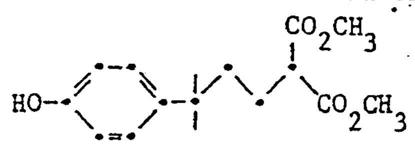 |
| 7 | 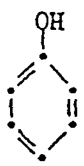 (94) | 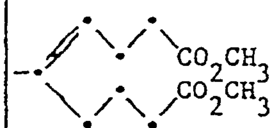 (45.6) | 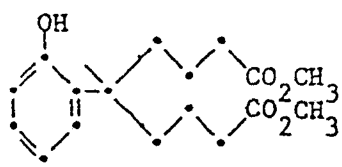 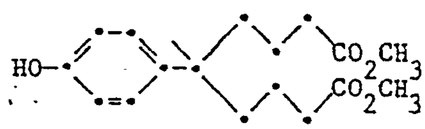 |
| 8 | 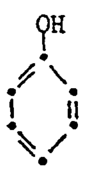 (37.8) | 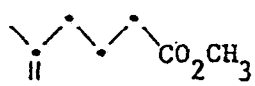 (28.4) | 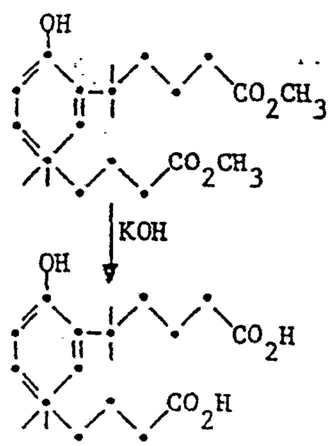 |

**Tabelle 1a:** (Fortsetzung)

| Beispiel | Phenol (Teile) | Alkylierungsmittel (Teile) | Produkte |
|---|---|---|---|
| 9 | 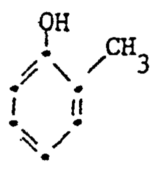 (108) | 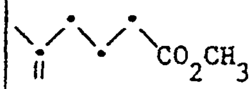 (56.8) | 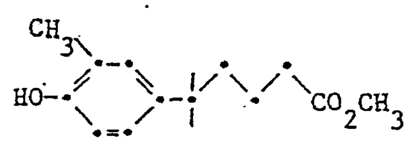 |
| 10 | 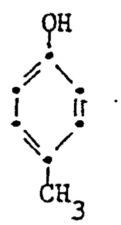 (108) | 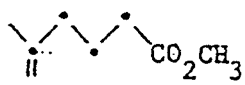 (56.8) | 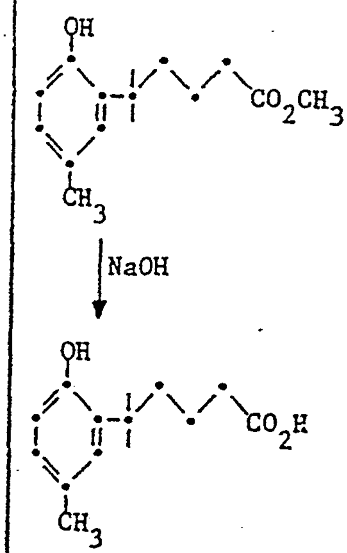  |
| 11 | 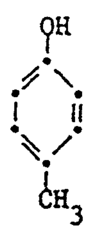 (108) | 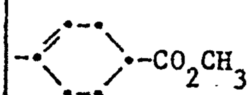 (61.6) | 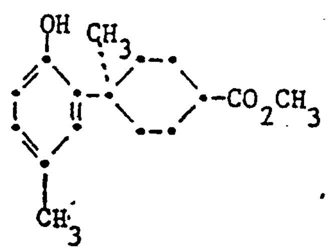 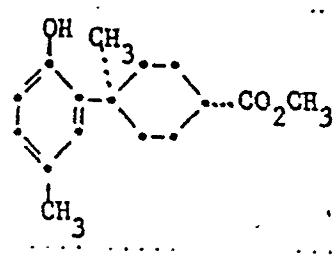 |

**Tabelle 1a.** (Fortsetzung)

| Beispiel | Phenol (Teile) | Alkylierungsmittel (Teile) | Produkte |
|---|---|---|---|
| 12 | 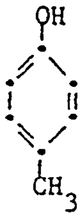 (540) | 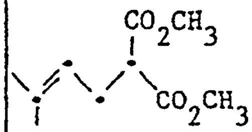 (100) | 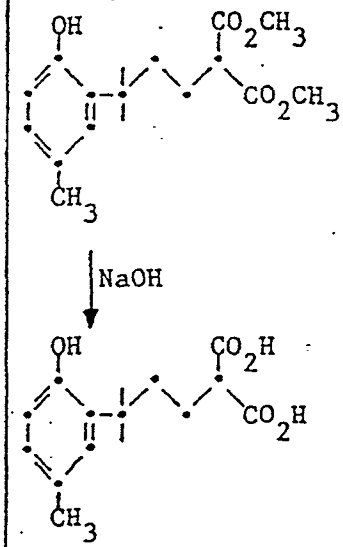 |
| 13 | 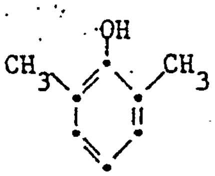 (122) | 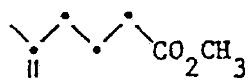 (28.4) | 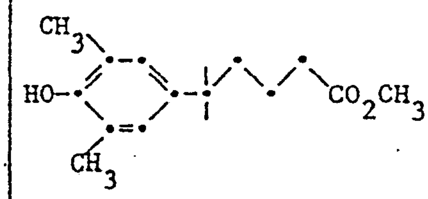 |
| 14 | 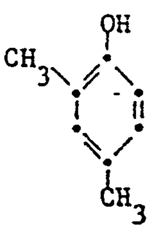 (122) | 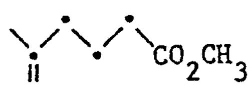 (28.4) | 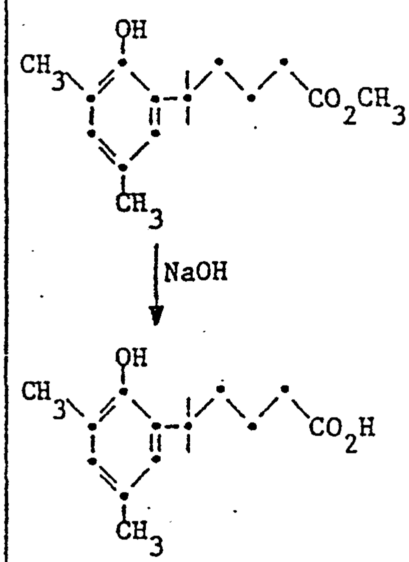 |

Tabelle 1a: (Fortsetzung)

| Beispiel | Phenol (Teile) | Alkylierungsmittel (Teile) | Produkte |
|---|---|---|---|
| 15 | 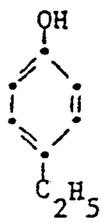 (122) | 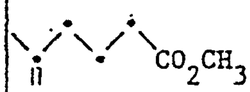 (47) | 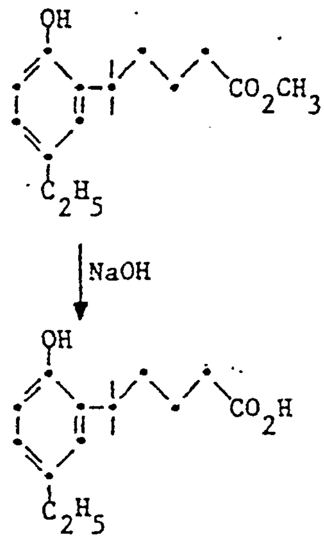 |
| 16 | 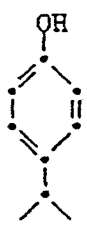 (136) | 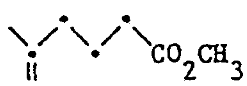 (47) | 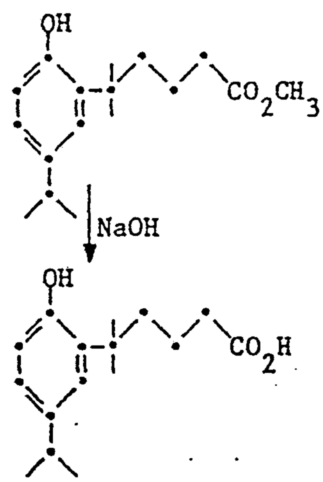 |

**Tabelle 1a:** (Fortsetzung)

| Beispiel | Phenol (Teile) | Alkylierungsmittel (Teile) | Produkte |
|---|---|---|---|
| 17 | 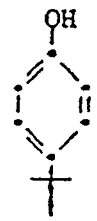 (75) | 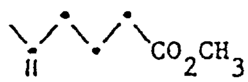 (28.4) | 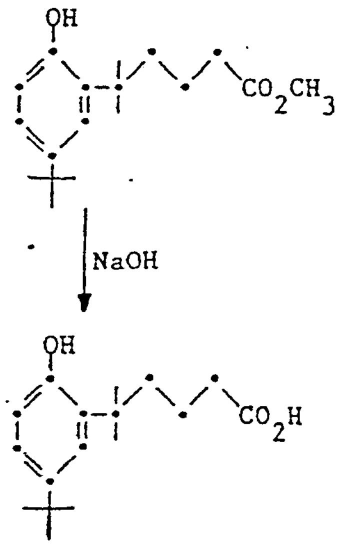 |
| 18 | 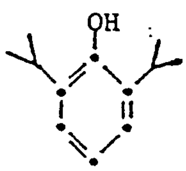 (71.2) | 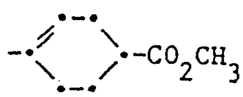 (61.6) | 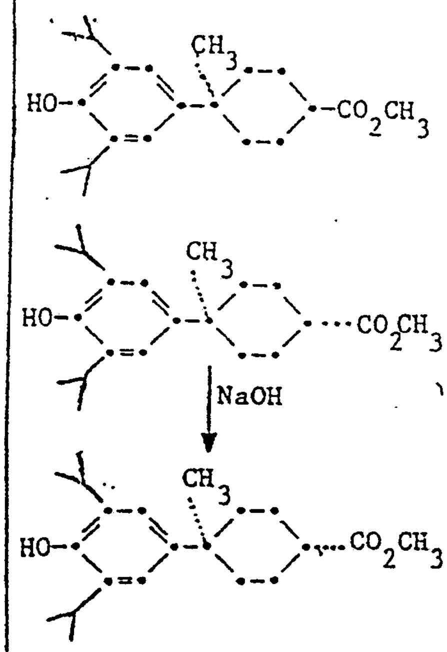 |

**Tabelle 1a:** (Fortsetzung)

| Beispiel | Phenol (Teile) | Alkylierungsmittel (Teile) | Produkte |
|---|---|---|---|
| 19 | 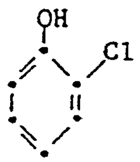 (64) | 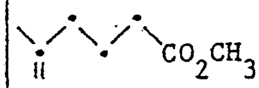 (14.2) | 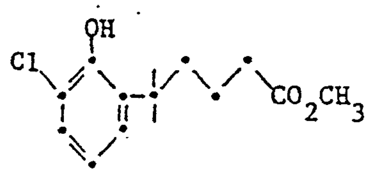 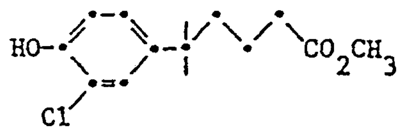 |
| 20 | 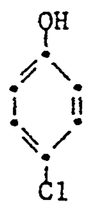 (64) | 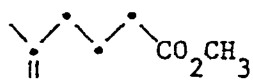 (14.2) | 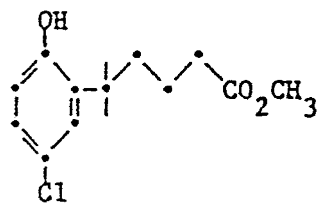 |

**Tabelle 1b:**

| Beispiel | Katalysator (Teile) | Reaktionstemp. (°C) | Reaktionszeit (Std.) | K.P./Druck (°C/mb) | F.P. (°C) | Summenformel | Analysedaten (%) gef. ber. | | |
|---|---|---|---|---|---|---|---|---|---|
| | | | | | | | C | H | Cl |
| 3 | $Al(OPh)_3$ als Al (0.3) | 175 | 40 | 190-250/13 | 125-7 | $C_{15}H_{20}O_3$ | 72.80<br>72.55 | 8.12<br>8.12 | -<br>- |
| 4 | Fulmont 237® (5.0) | 110 | 18 | 196-200/0.5 | 129-31 | $C_{17}H_{22}O_5$ | 66.65<br>66.65 | 7.36<br>7.24 | -<br>- |
| 5 | Fulmont 237® | 140 | 48 | 152-62/0.3 | | $C_{18}H_{28}O_3$ | 73.66<br>73.93 | 9.35<br>9.65 | -<br>- |
| 6 | Fulmont 237® (10) | 130 | 18 | 200/0.13 | | $C_{16}H_{22}O_5$ | 65.37<br>65.29 | 7.89<br>7.53 | -<br>- |
| 7 | Fulmont 237® (5.0) | 140 | 20 | 210-6/0.13 | | $C_{18}H_{26}O_5$ | 67.05<br>67.08 | 8.26<br>8.07 | - |
| 8 | Fulmont 237® (5.0) | 125 | 20 | 200-10/0.3 | 61-3 | $C_{22}H_{34}O_5$ | 70.03<br>69.81 | 8.80<br>9.05 | - |
| | | | | | 132-4 | $C_{20}H_{30}O_5$ | 68.88<br>68.55 | 8.61<br>8.63 | -<br>- |

**Tabelle 1b:** (Fortsetzung)

| Bei-spiel | Katalysator (Teile) | Reaktions-temp. (°C) | Reaktions-zeit (Std.) | K.P./Druck (°C/mb) | F.P. (°C) | Summen-formel | Analysedaten (%) gef. ber. C | H | Cl |
|---|---|---|---|---|---|---|---|---|---|
| 9 | Fulmont 237® (5.0) | 125 | 24 | 152-69/0.3 | 56-7 | $C_{15}H_{22}O_3$ | 72.21<br>71.97 | 8.87<br>8.86 | - |
| 10 | Fulmont 237® (5.0) | 150 | 20 | 144-6/1.3 | | $C_{15}H_{22}O_3$ | 72.03<br>71.97 | 8.74<br>8.86 | -<br>- |
| | | | | | 101-3 | $C_{14}H_{20}O_3$ | 71.08<br>71.16 | 8.67<br>8.53 | - |
| 11 | Fulmont 237® (5.0) | 150 | 6 | 160-70/0.3 | 141-3 | $C_{16}H_{23}O_3$ | 72.95<br>73.25 | 8.75<br>8.45 | -<br>- |
| | | | | | 134-6 | $C_{16}H_{22}O_3$ | 72.99<br>73.25 | 8.75<br>8.45 | -<br>- |
| 12 | Fulmont 237® (25) | 125 | 18 | | | | | | |
| | | | | | 115-6 | $C_{15}H_{20}O_5 \cdot H_2O$ | 60.88<br>60.39 | 7.58<br>7.43 | -<br>- |

**Tabelle 1b:** (Fortsetzung)

| Bei-spiel | Katalysator (Teile) | Reaktions-temp. (°C) | Reaktions-zeit (Std.) | K.P./Druck (°C/mb) | F.P. (°C) | Summen-formel | Analysedaten (%) gef. ber. C | H | Cl |
|---|---|---|---|---|---|---|---|---|---|
| 13 | Fulmont 237® (10) | 120 | 24 | 156-64/0.3 | 40-2 | $C_{16}H_{24}O_3$ | 72.70<br>72.69 | 9.01<br>9.15 | -<br>- |
| 14 | Fulmont 237® (10) | 120 | 24 | 148-52/0.3 | | | | | |
| | | | | | 159-62 | $C_{15}H_{22}O_3$ | 71.98<br>71.97 | 8.86<br>8.86 | -<br>- |
| 15 | Fulmont 237® (5.0) | 150 | 18 | 168-78/0.65 | | | | | |
| | | | | | 112-5 | $C_{15}H_{22}O_3$ | 71.84<br>71.97 | 8.88<br>8.86 | -<br>- |
| 16 | Fulmont 237® (5.0) | 150 | 18 | 171-4/0.65 | | $C_{17}H_{26}O_3$ | 73.62<br>73.35 | 9.37<br>9.41 | -<br>- |
| | | | | | 142-5 | $C_{16}H_{24}O_3$ | 72.97<br>72.69 | 9.30<br>9.15 | -<br>- |

**Tabelle 1b:** (Fortsetzung)

| Bei-spiel | Katalysator (Teile) | Reaktions-temp. (°C) | Reaktions-zeit (Std.) | K.P./Druck (°C/mb) | F.P. (°C) | Summen-formel | Analysedaten (%) gef. ber. C | H | Cl |
|---|---|---|---|---|---|---|---|---|---|
| 17 | Fulmont 237® (5.0) | 125 | 20 | | | | | | |
| | | | | | 111-4 | $C_{17}H_{26}O_3$ | 73.23<br>73.35 | 9.49<br>9.41 | -<br>- |
| 18 | Fulmont 237® (5.0) | 150 | 7 | 164-6/0.65 | 113-5 | $C_{21}H_{32}O_3$ | 75.64<br>75.86 | 9.67<br>9.70 | -<br>- |
| | | | | | 161-3 | $C_{20}H_{30}O_3$ | 75.54<br>75.43 | 9.64<br>9.49 | -<br>- |
| 19 | Fulmont 237® (5.0) | 125 | 24 | 214-8/13 | 48-50 | $C_{14}H_{19}ClO_3$ | 62.01<br>62.10 | 7.18<br>7.07 | 13.36<br>13.09 |
| 20 | Fulmont 237® (5.0) | 150 | 24 | 172-5/0.5 | 80-2 | $C_{14}H_{19}ClO_3$ | 62.15<br>62.10 | 7.10<br>7.07 | 12.95<br>13.09 |

**Beispiel 21:**

a) 12,5 Teile Methyl-5-(2-hydroxy-5-methylphenyl)-5-methylhexanoat (hergestellt wie in Beispiel 10 beschrieben), 14,2 Teile Methyl-5-methyl-hex-5-enoat und 1,0 Teile p-Toluolsulfonsäure werden 8 Tage auf dem Wasserbad erhitzt. Die Reaktionslösung wird mit Ether verdünnt, mit 2N Natronlauge gewaschen, mit Wasser ausgeschüttelt und eingeengt. Das zurückbleibende Oel wird fraktioniert destilliert. Die Fraktion mit dem $K.P._{0,4}$ 200°C bis 210°C enthält das Produkt Bis-2,6-(5-methoxy-carbonyl-2-methylpent-2-yl)-4-methylphenol. Aus Petrolether vom K.P. 40°C bis 60°C umkristallisiert erhält man ein Produkt mit dem $K.P._{0,4}$ 206°C bis 210°C und mit dem F.P. 55°C bis 57°C.

Analyse: C H
berechnet 70,38 9,24
gefunden 70,25 9,01

b) Durch Hydrolyse von Bis-2,6-(2-methoxycarbonyl-2-methylpent-2-yl)-4-methylphenol (Beispiel 21a) mit wässriger Kalilauge lässt sich die Dicarbonsäure herstellen. Das Produkt wird aus etherhaltigem Petrolether vom K.P. 40°C bis 60°C umkristallisiert und hat einen F.P. von 138°C bis 140°C.

Analyse: C H
berechnet 69,20 8,85
gefunden 69,38 9,13

**Beispiel 22:** 37,8 Teile

Bis-2,4-(5-methoxycarbonyl-2-methylpent-2-yl)-phenol (Beispiel 8), 28,4 Teile Methyl 5-methylhex-5-enoat und 3,0 Teile p-Toluolsulfonsäure werden 48 Stunden auf dem Wasserbad erhitzt. Das Rohprodukt wird wie in Beispiel 21 a) beschrieben aufgearbeitet und man erhält bei der fraktionierten Destillation Tris-2,4,6-(5-methoxycarbonyl)-2-methylpent-2-yl)-phenol vom $K.P._{0,3}$ 230°C bis 242°C, das mit etwa 10% des Bis-2,4-diesters verunreinigt ist. Der rohe Triester wird in Kalilauge hydrolysiert und man erhält reines Tris-2,4,6-(5-carboxy-2-methylpent-2-yl)-phenol, das nach Umkristallisstion aus Wasser einen F.P. von 156°C bis 160°C besitzt.

Analyse: C H
berechnet 67,76 8,84
gefunden 67,61 8,89

**Beispiel 23:**

a) 94 Teile Phenol, 35,4 Teile Dimethyl-[3-methyl-but-2-en-1-yl]-phos-phonat und 5,0 Teile Fulmont 237® werden bei 125°C 15 Stunden lang gerührt.

Die Reaktionsmischung wird abfiltriert und der Ueberschuss an Phenol im Vakuum abdestillitiert. Das zurückbleibende Oel wird mit Ether/Petrolether (K.P. 40°C bis 60°C) aufgenommen und bei 0°C stehengelassen. Aus dieser Lösung kristallisiert Dimethyl-3-(4-hydroxyphenyl)-3-methyl-butylphosphonat vom F.P. 98°C bis 101°C.

Analyse: C H P
berechnet 57,56 7,75 11,07
gefunden 57,41 7,83 11,38

b) 3,0 Teile Dimethyl-3-(4-hydroxyphenyl)-3-methylbutylphosphonat (Beispiel 23 a) und 30 Teile 48%ige Bromwasserstoffsäure werden 1 Stunde lang auf dem Wasserbad erhitzt und die flüchtigen Bestandteile anschliessendbei 80°C im Vakuum entfernt. Der feste Rückstand wird aus Wasser umkristallisiert und man erhält 3-(4-Hydroxyphenyl)-3-methylbutylphosphonsäure vom Schmelzpunkt 192°C bis 195°C.

Analyse: C H P
berechnet 54,32 6,09 12,34
gefunden 54.01 7,06 12,52

**Beispiel 24:** 94 Teile Phenol, 26 Teile

Diethyl-2-ethoxycarbonyl-5-methylhex-4-en-2-phosphonat und 5,0 Teile Fulmont 237® werden für 24 Stunden bei 110°C gerührt und anschliessend filtriert. Das überschüssige Phenol wird abgezogen. Durch Kurzwegdestillation des Rückstands erhält man ein Gemisch aus Diethyl-5-(2-hydroxyphenyl)-2-eth-oxycarbonyl-5-methylhexan-2-phosphonat und aus Diethyl-5-(4-hydroxy-phenyl)-2-ethoxycarbonyl-5-methylhexan-2-phosphonat.

Analyse: C H P
berechnet 60,15 8,27 7,51
gefunden 60,14 8,16 7,73

10 Teile dieses Gemisches werden durch präparative H.P.L.C. aufgetrennt Als Trägermaterial werden 300 Teile

Kieselerde verwendet, die vorher mit 6% Ethanol in Hexan desaktiviert worden war. Das zu trennende Gemisch wird in einer einzigen Injektion auf die Säule gegeben. Das o-substituierte Isomer erhält man durch Eluieren mit 5% Ethanol in Hexan. Nach dem Umkristallisieren aus etherhaltigem Petrolether (K.P. 40°C bis 60°C) schmilzt das Produkt bei 78°C bis 80°C.

Das p-substituierte Isomere wird anschliessend mit Dichlormethan eluiert und ebenfalls aus etherhaltigem Petrolether umkristallisiert. Das Produkt schmilzt bei 86°C bis 89°C.

Das Ausgangsmaterial Diethyl-2-ethoxycarbonyl-5-methylhex-4-en-2-phosphonat wird nach dem folgenden Verfahren hergestellt:

4,8 Teile Natriummetall werden in 200 Teilen absolutem Ethanol gelöst. Zu dieser Lösung tropft man 50 Teile Diethyl-l-ethoxycarbonylethylphosphonat. Die Reaktionslösung wird gerührt und es werden über einen Zeitraum von 1 Stunde 31,3 Teile 2-Brom-2-methylbut-3-en zugefügt. Nach der Addition wird weitere 2 Stunden bei 60°C gerührt. Nach dem Abkühlen wird das ausgefallene Natriumbromid abfiltriert und das überschüssige Ethanol unter Vakuum abgezogen. Der ölige Rückstand wird in Ether aufgenommen, mit Wasser gewaschen und wiederum eingeengt. Der Rückstand wird fraktioniert destilliert und man erhält Diethyl-2-ethoxycarbonyl-4-methylhex-4-en-2-phosphonat vom K.P.$_{0,3}$ 126°C bis 134°C.

Analyse: C H P
berechnet 55,08 8,85 9,83
gefunden 54,87 9,17 9,84

**Beispiel 25:** 24,4 Teile 2,6-Xylenol, 98 Teile 98%ige Schwefelsäure,

18 Teile Wasser und 17,8 Teile Dimethyl-[3-methyl-but-2-en-1-yl]-phospho-nat werden 4 Tage bei Raumtemperatur gerührtund anschliessend in 1000 Teile Wasser gegossen. Es scheidet siche in Oel ab, das mit Ether augenommen wird. Die Etherphase wird abgetrennt, mit Wasser, Kaliumbicarbonatlösung und nochmals mit Wasser gewaschen. Nach Entfernung des Ethers wird das zurückbleibende Oel fraktioniert destilliert. Die Fraktion vom K.P.$_{0,4}$ 200°C bis 205°C enthältDimethyl-3-(4-hydroxy-3,5-dimethyl-phenyl)-3-methylbut-1-ylphosphonat, das nach Umkristallisation aus etherhaltigem Petrolether (K.P. 40°C bis 60°C) einen Schmelzpunkt von 80°C bis 91°C besitzt.

Analyse: C H P
berechnet 59,98 8,39 10,31
gefunden 59,81 8,67 10,34

**Beispiel 26:**

a) Auf die gleiche Art und Weise wie in Beispiel 25 beschrieben stellt man Dimethyl 3-(4-hydroxy-3,5-diisopropylphenyl)-3-methylbut-1-yl-phosphonat her, indem man 35,6 Teile 2,6-Diisopropylphenol anstelle von 2,6-Xylenol verwendet. Das Produkt befindet sich in der Fraktion mit dem K.P.$_{0,3}$ 180°C bis 204°C und schmilzt bei 81°C bis 83°C.

Analyse: C H P
berechnet 64,04 9,27 8,70
gefunden 64,22 9,29 9,00

b) Dimethyl 3-(4-hydroxy-3,5-diisopropylphenyl)-3-methylbut-1-yl-phosphonat (Beispiel 26 a) wird wie in Beispiel 23b beschrieben hydrolysiert. Man erhält 3-(4-Hydroxy-3,5-diisopropylphenyl)-3-methyl-but-1-ylphosphonsäure vom Schmelzpunkt 179°C bis 182°C (umkristallisiert aus Tetrachlorkohlenstoff der etwas Petrolether enthält).

Analyse: C H P
berechnet 58,95 8,95 8,95
gefunden 59,58 8,55 8,86

**Beispiel 27:** 12,8 Teile 2,6-Dichlorphenol, 14,2 Teile

Methyl-5-methyl-hex-5-enoat,130 Teile 98%ige Schwefelsäure und 24 Teile Methanol werden 24 Stunden auf dem Wasserbad gerührt. Die Reaktionslösung wird wie in Beispiel 25 beschrieben aufgearbeitet. Die Fraktion vom K.P.$_{0,5}$ 140°C bis 160°C enthält Methyl-5-(3,5-dichlor-4-hydroxyphenyl)-5-methylhexanoat vom Schmelzpunkt 101°C bis 103°C.

Analyse: C H Cl
berechnet 55,09 5,94 23,24
gefunden 55,18 6,04 23,07

**Beispiele 28 bis 43:**

Weitere Beispiele für die funktionelle Alkylierung von Phenolen werden in den folgenden Tabellen 2a und 2b gegeben.
In Tabelle 2a sind Verbindungen der Formel I sowie die Ausgangsprodukte zu deren Herstellung aufgelistet, während Tabelle 2b Analysedaten, Schmelz- oder Siedepunkte der Verbindungen enthält, sowie Einzelheiten zur Reaktionsdurchführung.

**Tabelle 2a:**

| Beispiel | Phenol (Teile) | Alkylierungsmittel (Teile) | Produkte |
|---|---|---|---|
| 28 | 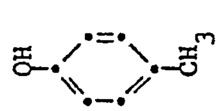 (108) | 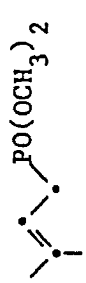 (23.4) | 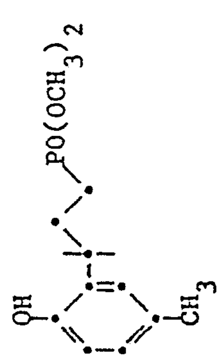 |
| 29 | 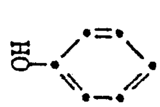 (94) | 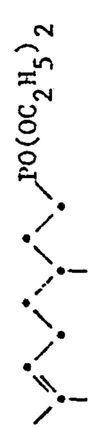 (27.6) | 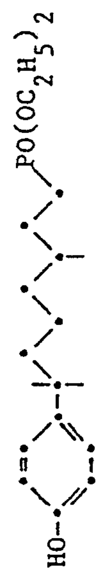 |
| 30 | 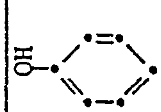 (94) | 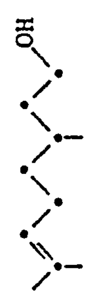 (78) | 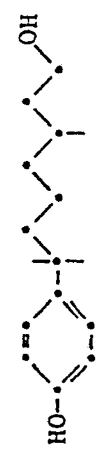 |
| 31 | 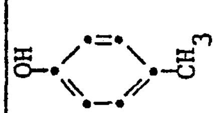 (108) | 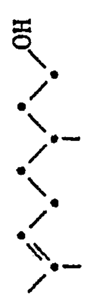 (31.2) | 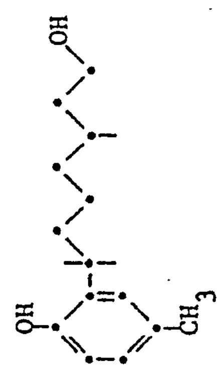 |

**Tabelle 2b:** (Fortsetzung)

| Beispiel | Phenol (Teile) | Alkylierungsmittel (Teile) | Produkte |
|---|---|---|---|
| 32 | 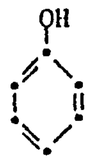 (94) | 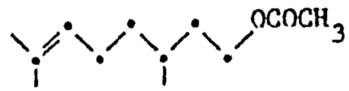 (88) | 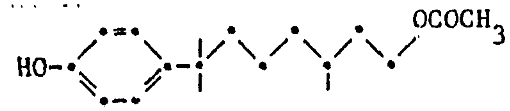 |
| 33 | 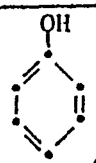 (47) | 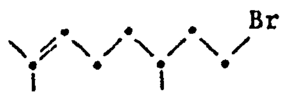 (11.0) | 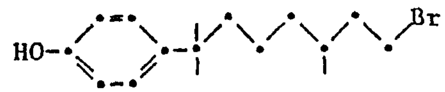 |
| 34,a | 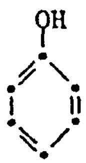 (47) | 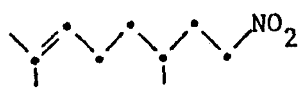 (7.5) | 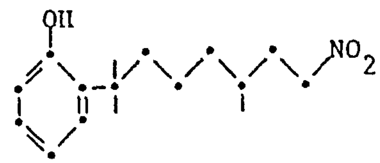 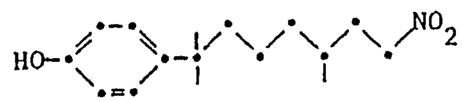 |
| 35 | 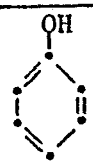 (94) | 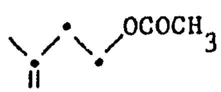 (25.6) | 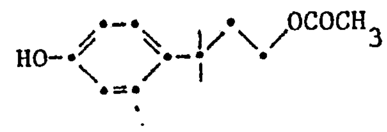 |

Tabelle 2a: (Fortsetzung)

| Beispiel | Phenol (Teile) | Alkylierungsmittel (Teile) | Produkte |
|---|---|---|---|
| 36 | 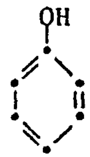 (94) | 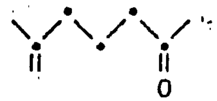 (25.2) | 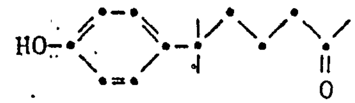 |
| 37 | 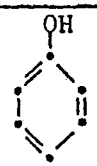 (94) | 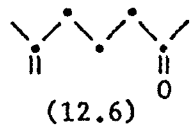 (12.6) | 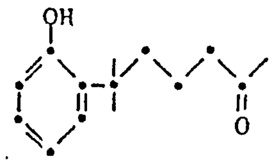 |
| 38 | 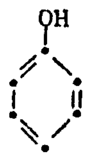 (94) | 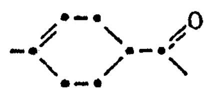 (13.8) | 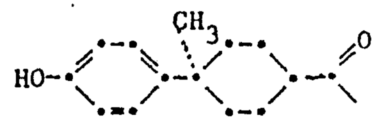 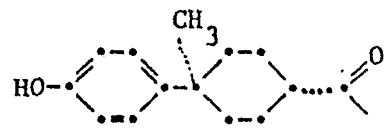 |

**Tabelle 2a:** (Fortsetzung)

| Beispiel | Phenol (Teile) | Alkylierungsmittel (Teile) | Produkte |
|---|---|---|---|
| 39 | 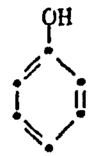 (94) | 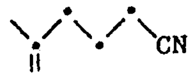 (16.0) | 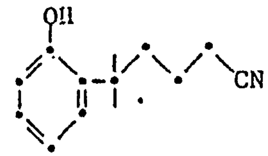 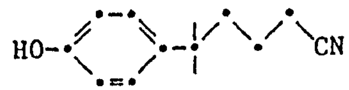 |
| 40 | 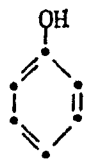 (94) | 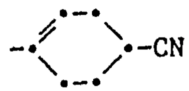 (24.2) | 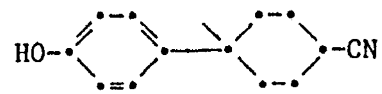 |
| 41 | 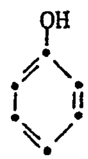 (94) | 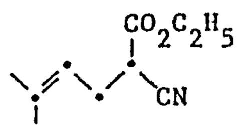 (18.1) | 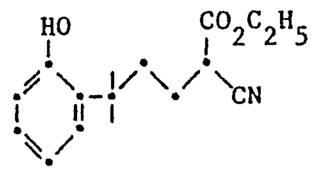 |

Tabelle 2a: (Fortsetzung)

| Beispiel | Phenol (Teile) | Alkylierungsmittel (Teile) | Produkte |
|---|---|---|---|
| 42 | 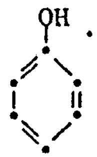 (94) | 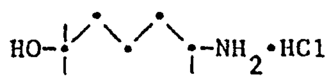 (18.1) | 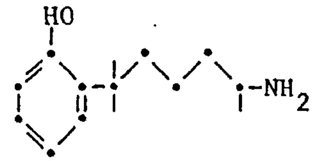 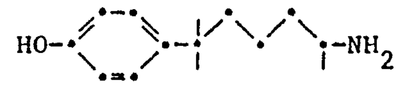 |
| 43 | 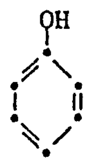 (94) | 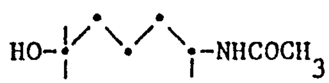 (18.7) | 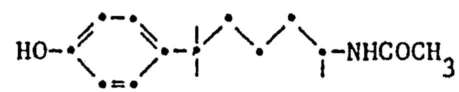 |

**Tabelle 2b:**

| Beispiel | Katalysator (Teile) | Reaktionstemp. (°C) | Reaktionszeit (Std.) | K.P./Druck (°C/mb) | F.P. (°C) | Summenformel | Analysedaten (%) gef./ber. C | H | P |
|---|---|---|---|---|---|---|---|---|---|
| 28 | Fulmont 237® (5.0) | 120 | 26 | | 119-21 | $C_{14}H_{23}O_4P$ | 58.96<br>58.94 | 8.07<br>8.07 | 10.72<br>10.52 |
| 29 | Fulmont 237® (5.0) | 110 | 18 | 200/0.13<br>Kurzwegdest. | | $C_{20}H_{35}O_4P$ | 64.83<br>64.86 | 9.63<br>9.46 | 8.19<br>8.37 |
| 30 | Fulmont 237® (5.0) | 110-20 | 24 | 178-80/0.065 | | $C_{16}H_{26}O_2$ | 76.60<br>76.75 | 10.68<br>10.47 | |
| 31 | Fulmont 237® (5.0) | 125 | 18 | 175-82/0.3 | | $C_{17}H_{28}O_2$ | 77.22<br>78.24 | 10.67<br>10.41 | |
| 32 | Fulmont 237® (5.0) | 110 | 24 | 175-90/0.3 | | $C_{18}H_{28}O_3$ | 74.00<br>73.93 | 9.90<br>9.65 | |
| 33 | Fulmont 237® (2.5) | 110 | 6 | 166-70/0.13 | | $C_{16}H_{25}BrO$ | 61.29<br>61.34 | 8.00<br>7.98 | |

**Tabelle 2b:** (Fortsetzung)

| Beispiel | Katalysator (Teile) | Reaktionstemp. (°C) | Reaktionszeit (Std.) | K.P./Druck (°C/mb) | F.P. (°C) | Summenformel | Analysedaten (%) gef. ber. C | H | N |
|---|---|---|---|---|---|---|---|---|---|
| 34a | Fulmont 237® (2.5) | 110-20 | 4 | 186-200/0.07 | | $C_{16}H_{25}NO_3$ | 68.59<br>68.79 | 9.02<br>9.02 | 5.01<br>5.01 |
| | | | | | | $C_{16}H_{25}NO_3$ | 68.81<br>68.79 | 9.28<br>9.02 | 4.93<br>5.01 |
| 35 | Fulmont 237® (5.0) | 150 | 2 | 154-60/0.65 | | $C_{13}H_{18}O_3$ | 71.33<br>70.24 | 8.24<br>8.16 | |
| 36 | Fulmont 237® (5.0) | 110 | 18 | 150/0.3 Kurzwegdest. | | $C_{14}H_{20}O_2$ | 76.70<br>76.33 | 9.08<br>9.15 | |
| 37 | $Al(OPh)_3$ als Al (1.0) | 125 | 40 | | | $C_{14}H_{20}O_2$ | 76.25<br>76.33 | 9.03<br>9.15 | |
| 38 | Fulmont 237® (5.0) | 110 | 3 | 188-94/0.8 | 129-31 | $C_{15}H_{20}O_2$ | 77.82<br>77.55 | 8.84<br>8.68 | |
| | | | | | 126-8 | $C_{15}H_{20}O_2$ | 76.95<br>77.55 | 8.75<br>8.68 | |

**Tabelle 2b:** (Fortsetzung)

| Beispiel | Katalysator (Teile) | Reaktionstemp. (°C) | Reaktionszeit (Std.) | K.P./Druck (°C/mb) | F.P. (°C) | Summenformel | Analysedaten (%) gef. ber. C | H | N |
|---|---|---|---|---|---|---|---|---|---|
| 39 | Fulmont 237® (5.0) | 150 | 20 | 218/13 | 90-2 | $C_{13}H_{17}NO$ | 77.08<br>76.81 | 8.56<br>8.43 | 6.65<br>6.89 |
| | | | | | | $C_{13}H_{17}NO$ | 77.07<br>76.81 | 8.50<br>8.43 | 6.88<br>6.89 |
| 40 | Fulmont 237® (5.0) | 165 | 48 | 252-8/13 | | $C_{14}H_{17}NO$ | 78.48<br>78.10 | 8.18<br>7.96 | 6.08<br>6.51 |
| 41 | Fulmont 237® (5.0) | 110 | 20 | 200/0.13 Kurzwegdest. | | $C_{16}H_{21}NO_3$ | 70.07<br>69.79 | 8.24<br>7.69 | 4.68<br>5.09 |
| 42 | Fulmont 237® (5.0) | 110 | 20 | 198/16 | 96-8 | $C_{14}H_{23}NO$ | 76.00<br>75.97 | 10.22<br>10.47 | 6.38<br>6.33 |
| | | | | | 119-22 | $C_{14}H_{23}NO$ | 76.27<br>75.97 | 10.72<br>10.47 | 6.28<br>6.33 |
| 43 | Fulmont 237® (5.0) | 110 | 8 | 217-25/0.07 | 105-7 | $C_{16}H_{25}NO_2$ | 73.07<br>72.97 | 9.45<br>9.57 | 5.30<br>5.32 |

**Beispiel 34b:**

Herstellung von 3,7-Dimethyl-1-nitrooct-6-en (Alkylierungsmittel in Beispiel 34a):

Zu einer Lösung bestehend aus 13,3 Teilen wasserfreiem Phloroglucin, 13,3 Teilen Harnstoff, 12,0 Teilen Natriumnitrit in 200 Teilen Dimethylformamid werden 21,9 Teile Citronellylbromid zugetropft. Die Temperatur wird dabei unter 25° C gehalten. Nach der Addition wird 48 Stunden ausgerührt und die Reaktionsmischung anschliessend in Wasser gegossen. Die organische Phase wird abgetrennt und der wässrige Rückstand mehrmals mit Ether ausgeschüttelt. Die vereinigten Etherphasen werden mit Wasser ausgeschüttelt. Nach dem Verdampfen des Ethers wird der Rückstand fraktioniert destilliert.

Der Vorlauf bis zum $K.P._{16}$ 110° C enthält 4,8 Teile Citronellol, während die zweite Fraktion vom $K.P._{16}$ 130° C bis 132° C 8,2 Teile des Produkts 3,7-Dimethyl-1-nitrooct-6-en enthält.

Analyse: C H N
berechnet 64,83 10,30 7,56
gefunden 64,47 10,57 7,32

**Beispiel 44:** Durch eine Lösung aus 35,0 Teilen

Methyl-5-(4-hydroxy-3-methylphenyl)-5-methylhexanoat(Beispiel 9), 2,0 Teilen 98%iger Schwefelsäure und 200 Teilen Toluol wird 2 Stunden lang bei 75° C Isobutylen geleitet. Anschliessend wird die Reaktionslösung mit 10%iger Natronlauge und mit Wasser gewaschen, das Toluol abgezogen und der Rückstand fraktioniert destilliert. Man erhält Methyl-5-(3-tert.butyl-4-hydroxy-5-methylphenyl)-5-methylhexanoat vom $K.P._{0,3}$ 168° C bis 172° C.

Analyse: C H
berechnet 74,47 9,87
gefunden 74,71 10,08

**Beispiel 45:** Nach der gleichen Methode wie in Beispiel 44 erhält

man Methyl-5-(3-tert.butyl-2-hydroxy-5-isopropylphenyl)-5-methyl-hexanoat vom $K.P._{0,3}$ 152° C bis 154° C, wenn man anstelle von Methyl-5-(4-hydroxy-3-methylphenyl)-5-methylhexanoat das Reaktionsprodukt von Beispiel 16 Methyl-5-(2-hydroxy-5-isopropylphenyl)-5-methylhexanoat verwendet.

Analyse: C H
berechnet 75,41 10,25
gefunden 75,58 10,31

**Beispiel 46:** 50 Teile Methyl-5-(2-hydroxy-5-methylphenyl)-5-methyl-

hexanoat, 30 Teile Isobutylen, 2,0 Teile p-Toluolsulfonsäure und 160 Teile Cyclohexan werden 24 Stunden in einem Autoklaven bei 100° C gerührt. Die Reaktionslösung wird mit 10%iger Natriumhydroxidlösung und anschliessend mit Wasser gewaschen und das Cyclohexan abgezogen. Das zurückbleibende Oel wird über eine Säule, die 500 Teile Kieselgel in Petrolether (K.P. 40° C bis 60° C) enthält, mit 5% Ether in Petrolether eluiert und man erhält reines Methyl-5-(3-tert.butyl-2-hydroxy-5-methylphenyl)-5-methylhexanoat mit dem $K.P._{0,13}$ 142° C bis 150° C.

Analyse: C H
berechnet 74,47 9,87
gefunden 74,94 9,91

Aus dem Ester kann durch alkalische Verseifung und anschliessendesUmkristallisieren aus Petrolether (K.P. 40° C bis 60° C) 5-(3-tert.Butyl-2-hydroxy-5-methylphenyl)-5-methylhexansäure vom F.P. 99° C bis 101° C erhalten werden.

Analyse: C H
berechnet 73,93 9,65
gefunden 73,35 9,85

**Beispiel 47:**

Methyl-5-(3,5-di-tert.butyl-4-hydroxyphenyl)-5-methyl-hexanoat wird in Analogie zu Beispiel 44 aus Methyl-5-(4-hydroxyphenyl)-5-methylhexanoat (Beispiel 1 a) und Isobutylen hergestellt. Das Produkt schmilzt nach dem

Umkristallisieren aus Petrolether (K.P. 40°C bis 60°C) zwischen 71°C und 72°C.
Analyse: C H
berechnet 75,81 10,41
gefunden 75,96 10,64

**Beispiel 48:** Methyl-5-(3,5-di-tert.butyl-2-hydroxyphenyl)-5-methylhexanoat wird in Analogie zu Beispiel 46 hergestellt, indem man Methyl-5-(5-tert.butyl-2-hydroxyphenyl)-5-methylhexanoat (Beispiel 17) mit Isobutylen umsetzt. Das Produkt siedet beim K.P.$_{0,7}$ 152°Cbis 158°C
Analyse: C H
berechnet 75,81 10,41
gefunden 75,78 10,47

**Beispiel 49:** Durch eine Lösung aus 20 Teilen cis- und trans-4-Methoxy-carbonyl-1-(p-hydroxyphenyl)-1-methylcyclohexan (Beispiel 2), 2,0 Teilen 98%iger Schwefelsäure und 50 Teilen Benzol wird bei 70°C für 2 Stunden Isobutylengas geleitet. Nach dem Abkühlen wird mit Ether verdünnt, mit Natriumbicarbonatlösung gewaschen, und der Ether abgezogen. Das zurückbleibende Oel wird mit Petrolether (K.P. 40°C bis 60°C) versetzt. Die zurückbleibenden Kristalle bestehen aus cis-[4-Methoxycarbonyl-1-(3,5-di-tert.butyl-4-hydroxyphenyl)]-1-methylcyclohexan vom F.P. 152°C bis 155°C.
Analyse: C H
berechnet 76,62 10,07
gefunden 76,79 10,07
Aus den Mutterlaugen der oberen Kristallisation lässt sich eine zweite Kristallfraktion isolieren, die aus trans-[4-Methoxycarbonyl-1-(3,5-di-tert.butyl-4-hydroxyphenyl)]-1-methylcyclohexan besteht und nach dem Umkristallisieren aus Methanol zwischen 95°C und 97°C schmilzt.
Analyse: C H
berechnet 76,62 10,07
gefunden 76,90 10,02

**Beispiel 50:** 27,0 Teile trans-[4-Methoxycarbonyl-1-(3,5-di-tert.butyl-4-hydroxyphenyl)]-1-methylcyclohexan, 4,4 Teile Hexan-1,6-diolund 0,3 Teile Lithiumamid werden 24 Stunden bei 80°C und 13 mb gerührt. Dann werden 2,0 Teile Eisessig zugefügt, die Reaktionsmischung mit Ether verdünnt, mit Wasser gewaschen und dann wieder eingeengt. Der feste Rückstand wird aus Methanol umkristallisiert und man erhält Kristalle vom F.P. 120°C bis 122°C, die aus dem Diester der [4-Methyl-trans-(4-(3,5-di-tert.butyl-4-hydroxyphenyl)-cyclohexan-1-carbonsäure)] mit Hexan-1,6-diol bestehen.
Analyse: C H
berechnet 77,47 10,14
gefunden 77,80 9,84
Die folgenden Tabellen 3a und 3b enthalten weitere Beispiele für Verbindungen der Formel I, die in der gleichen Weise wie in Beispiel 50 beschrieben hergestellt werden.
Tabelle 3a enthält die Ausgangs- und Endprodukte der Reaktion, während in Tabelle 3b Analysedaten, Schmelz- oder Siedepunkte der Endprodukte sowie Einzelheiten über die Reaktionsdurchführung aufgelistet sind.

**Tabelle 3a:**

| Beispiel | Phenol (Teile) | Alkohol (Teile) | Produkte |
|---|---|---|---|
| 51 | 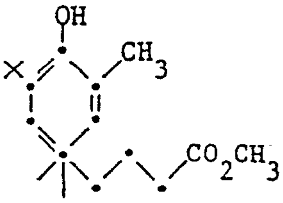 (10.2) | $HO(CH_2)_6OH$ (1.9) | 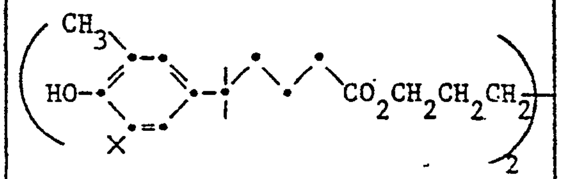 |
| 52 | 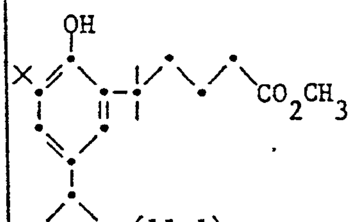 (11.1) | $HO(CH_2)_6OH$ (2.1) | 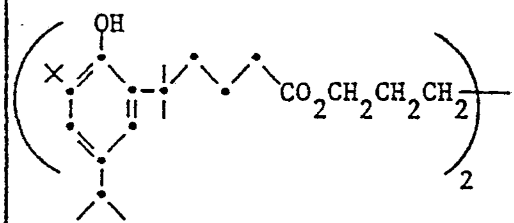 |
| | 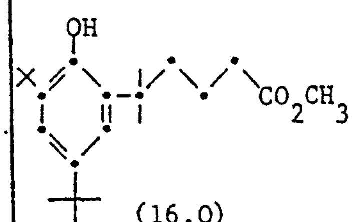 (16.0) | $HO(CH_2)_6OH$ (2.7) | 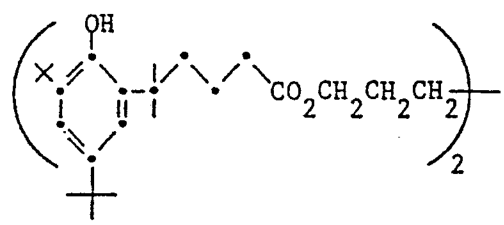 |

Tabelle 3b:

| Beispiel | Katalysator (Teile) | Reaktionstemp. (°C) | Reaktionszeit (Std.) | K.P./Druck (°C/mb) | Summenformel | Analysedaten (%) gef. ber. C | H |
|---|---|---|---|---|---|---|---|
| 51 | $LiNH_2$ (0.1) | 150 | 7 | 250/0.13 Kurzwegdest. | $C_{42}H_{66}O_6$ | 75.58<br>75.63 | 10.23<br>9.97 |
| 52 | $LiNH_2$ (0.1) | 150 | 7 | 250/0.7 Kurzwegdest. | $C_{46}H_{74}O_6$ | 75.23<br>75.40 | 10.26<br>10.26 |
| 53 | $LiNH_2$ (0.1) | 150 | 8 | 300-15/0.7 | $C_{48}H_{78}O_6$ | 76.98<br>76.75 | 10.24<br>10.47 |

**Beispiel 54:** 2,0 Teile 5-(3-tert.Butyl-2-hydroxy-5-methylphenyl)-5-methylhexansäure und 2,0 Teile Thionylchlorid werden in 25 Teilen Toluol bei Raumtemperatur 2 Stunden lang belassen. Anschliessend werden das Toluol und andere flüchtige Anteile bei 16 mb und Raumtemperatur abgezogen. Der Rückstand wird mit 10 Teilen 1,1,3,3-Tetra-methylbutylamin versetzt und 3 Stunden auf dem Wasserbad erhitzt. Anschliessend wird die Reaktionsmischung mit Ether verdünnt und sukzessive mit verdünnter Salzsäure, Wasser verdünnter Natronlauge und Wasser gewaschen. Der Ether wird abgedampft und der Rückstand aus Petrolether (K.P. 60°C bis 80°C) umkristallisiert. Man erhält Kristalle des Amids aus 5-(3-tert.Butyl-2-hydroxy-5-methylphenyl)-5-methyl-hexansäure und 1,1,3,3-Tetramethylbutylamin mit dem Schmelzpunkt 110°C bis 112°C.

Analyse: C H N
berechnet 77,37 11,24 3,47
gefunden 77,30 11,24 3,40

**Beispiel 55:** 9,0 Teile 5-(3-tert.Butyl-2-hydroxy-5-methylphenyl)-5-methylhexansäure (Beispiel 46) und 30,0 Teile n-Dodecylamin werden 24 Stunden bei 175°C gerührt. Das überschüssige n-Dodecylamin wird abdestilliert und es verbleibt das Amid der 5-(3-tert.Butyl-2-hydroxy-5-methylphenyl)-5-methylhexansäure mit n-Dodecylamin. Das Produkt besitzt einen $K.P._{0,3}$ 246°C und einen F.P. von 43°C bis 45°C.

Analyse: C H N
berechnet 78,43 11,54 3,05
gefunden 78,64 11,84 2,93

**Beispiel 56:** 14,6 Teile 5-(3-tert.Butyl-2-hydroxy-5-methylphenyl) 5 methylhexansäure (Beispiel 46) und 2,9 Teile Hexamethylendiamin werden für 24 Stunden auf 150°C erhitzt. Nach dem Abkühlen erhält man einen Feststoff, der aus Toluol umkristalliert wird. Man erhält das Bis-5-(3-tert.butyl-2-hydroxy-5-methylphenyl)-5-methylhexansäure amid des 1,6-Diaminohexans mit dem Schmelzpunkt von 139°C bis 142°C.

Analyse: C H N
berechnet 75,91 10,23 4,24
gefunden 76,17 10,08 4,19

**Beispiel 57:** 5,0 Teile5-(3-tert.Butyl-2-hydroxy-5-methylphenyl)-5-methylhexansäure (Beispiel 46) und 20 Teile Anilin werden 20 Stunden unter Rückfluss gekocht und dann in einen Ueberschuss von Salzsäure gegossen. Es scheidet sich ein Oel ab, das mit Ether extrahiert wird und sukzessive mit 2N Salzsäure, Natriumbicarbonatlösung und Wasser gewaschen wird. Nach dem Abdampfen des Ethers erhält man einen braunen Feststoff der säulenchromatographisch aufgetrennt wird. Man verwendet eine Säule, die 100 Teile Kieselgel in Petrolether (K.P. 40°C bis 60°C) suspendiert enthält. Eluiert wird mit einer 10%igen Lösung von Ether in Petrolether und man erhält N-Phenyl-5-methyl-5-(3-tert.butyl-2-hydroxy-5-methylphenyl)-hexamid, das nach dem Umkristallisieren aus etherhaltigem Petrolether (K.P. 40°C bis 60°C) zwischen 122°C und 124°C schmilzt.

Analyse: C H N
berechnet 78,43 9,05 3,81
gefunden 78,06 9,09 3,50

**Beispiel 58:** 30,4 Teile Methylsalicylat, 28,4 Teile Methyl-5-methyl-hex-5-enoat, 130 Teile 98%ige Schwefelsäure und 24 Teile Methanol werden 4 Tage bei Raumtemperatur gerührt. Die Reaktionsmischung wird wie in Beispiel 25 beschrieben aufgearbeitet und man erhält durch fraktionierte Destillation Methyl-2-hydroxy-5-(5-methoxycarbonyl-2-methylpent-2-yl)-benzoat vom $K.P._{0,3}$ 150°C bis 152°C.

Analyse: C H
berechnet 65,29 7,53
gefunden 65,27 7,65

und Methyl-2-hydroxy-3,5-bis-(5-methoxycarbonyl-2-methylpent-2-yl)-benzoat vom $K.P._{0,1}$ 220°C.

Analyse: C H

berechnet 66,03 8,31
gefunden 65,75 8,28

Durch alkalische Verseifung der oberen Ester lassen sich 5-(5-Carboxy-2-methylpent-2-yl)-2-hydroxybenzoesäure vom F.P. 136°C bis 138°C und 3,5-Bis-(5-carboxy-2-methylpent-2-yl)-2-hydroxybenzoesäure vom F.P. 161°C bis 163°C erhalten.

In Analogie zu Beispiel 58 kann Methyl-5-(6-amino-2-methylhept-2-yl)-2-hydroxybenzoat erhalten werden. In diesem Fall darf nicht destilliert werden, da das Material beim Erhitzen polymerisiert.

Analyse: C H N
berechnet 68,79 9,02 5,01
gefunden 67,93 9,10 4,90

Ebenso können erhalten werden: 5-(6-Amino-2-methylhept-2-yl)-hydroxybenzoesäure (F.P. 182°C bis 185°C),
Analyse: C H N
berechnet (Monohydrat) 63,58 8,89 4,94
gefunden 64,58 8,97 5,00

Methyl-2-hydroxy-5-(2-methyl-4-dimethylphosphono-but-2-yl)-benzoat (K.P.$_{0,1}$ 175°C),
Analyse: C H P
berechnet 54,54 7,02 9,38
gefunden 54,20 7,30 9,08

2-Hydroxy-5-(2-methyl-4-phosphono-but-2-yl)-benzoesäure (F.P. 125°C bis 135°C),
Analyse: C H P
berechnet 50,00 5,95 10,74
gefunden 49,07 6,25 11,97

Methyl-4-hydroxy-3-(5-methoxycarbonyl)-2-methylpent-2-yl)-benzoat (F.P. 80°C bis 82°C).
Analyse: C H
berechnet 65,29 7,53
gefunden 65,45 7,68

**Beispiel 59:** Zu einer Lösung aus 19,0 Teilen Wasser, 0,5 Teilen 98%iger Schwefelsäure und 0,2 Teilen Natriumdodecylbenzolsulfonat werden 25,0 Teile Methyl-5-(4-hydroxy-3-methylphenyl)-5-methylhexanoat (Beispiel 9) und 1,0 Teile Petrolether (K.P. 80°C bis 100°C) unter Rühren hinzugegeben. Die Mischung wird auf 80°C erwärmt und anschliessend werden 4,4 Teile einer 40%igen Formaldehydlösung über einen Zeitraum von 1 Stunde zugetropft.Die Reaktionslösung wird weitere 2 Stunden auf 80°C gehalten, um die Addition zu vervollständigen. Anschliessend werden 100 Teile Wasser zugegeben, die organische Phase mit Ether extrahiert, der Extrakt mit Natriumbicarhonatlösung und Wasser gewaschen und anschliessend eingeengt. Der Rückstand wird einer Kurzwegdestillation unterworfen. Man erhält 11,0 Teile Methyl-5-(4-hydroxy-3-methylphenyl)-5-methylhexanoat (bei einer Ofentemperatur $T_{0,7}$ 150°C)und Bis-[2-hydroxy-5-(5-methoxycarbonyl-2-methylpent-2-yl)-3-methylphenyl]-methan (bei einer Ofentemperatur $T_{0,7}$ 250°C)

Analyse: (2.Produkt) C H
berechnet 72,63 8,65
gefunden 72,55 8,69

**Beispiel 60:**
Bis-[2-hydroxy-3-(5-methoxycarbonyl-2-methylpent-2yl)-5-methylphenyl]-methan wird wie in Beispiel 59 angegeben aus Methyl-5-(2-hydroxy-5-methylphenyl)-5-methylhexanoat (Beispiel 10) hergestellt. Die Verbindung besitzt einen K.P.$_{0,07}$ von 284°C.

Analyse: C H
berechnet 72,63 8,65
gefunden 72,75 8,30

**Beispiel 61:** 12,2 Teile 2,6-Dimethylphenol, 5,3 Teile Citronellyl-n-butyletherund 1,0 Teile 70%ige Perchlorsäure werden in 30 Teilen Dichlormethan 3 Tage belassen. Anschliessend wird die Reaktionsmischung in Wasser gegossen und mit Ether extrahiert. Die vereinigten Etherphasen werden mit Natriumbicarbonatlösung und anschliessend mit Wasser gewaschen. Das Lösungsmittel wird abgezogen und der ölige Rückstand destilliert. Man erhält 1-n-Butyloxy-7-(4-hydroxy-3,5-dimethylphenyl)-3,7-dimethyloctan vom K.P.$_{0,8}$ 168°C bis 176°C.

Analyse: C H

berechnet 78,99 11,45
gefunden 79,05 11,52

**Beispiel 62:** 10,3 Teile 4-(5-Methoxycarbonyl-2-methylpent-2-yl)-phenol, 4,2 Teile Citronellyl-n-butylether und 0,5 Teile 70%iger Perchlorsäure werden 4 Tage in 25 Teilen Dichlormethan bei Raumtemperatur stehengelassen. Die Aufarbeitung erfolgt wie in Beispiel 61 beschrieben und man erhält bei der Destillation 2-(8-n-Butyloxy-2,6-dimethyloct-2-yl)-4-(5-methoxycarbonyl-2-methylpent-2-yl)-phenol vom $K.P._{0,8}$ 228°C bis 236°C.

Analyse: C H
berechnet 74,95 10,78
gefunden 75,02 11,08

**Beispiel 63:** 2,3 Teile 4-n-Decylphenol, 1,4 Teile Methyl-5-methylhex-5-enoat, 0,25 Teile 70%ige Perchlorsäure und 10 Teile Dichlormethan werden 12 Tage bei Raumtemperatur belassen. Es wird wie in Beispiel 61 beschrieben aufgearbeitet und man erhält bei der Destillation Methyl-5-(5-n-decyl-2-hydroxyphenyl)-5-methylhexanoat vom $K.P._{0,8}$ 160°C bis 167°C.

Analyse: C H
berechnet 76,55 10,71
gefunden 76,66 10,80

**Beispiel 64:** 5,0 Teile 2-Methyl-4-(5-methoxycarbonyl-2-methylpent-2-yl)-phenol, 2,4 Teile α-Methylstyrol und 0,5 Teile 70%iger Perchlorsäure werden in 25 Teilen Dichlormethan reagieren gelassen und aufgearbeitet wie in Beispiel 61 beschrieben.

Durch Destillation erhält man Methyl-5-(3-cumyl-4-hydroxy-5-methyl-phenyl)-5-methylhexanoat als Oel vom $K.P._{0,7}$ 210°C bis 214°C.

Analyse: C H
berechnet 78,78 8,89
gefunden 78,22 8,75

**Beispiel 65:** 27,2 Teile 2-Hydroxyacetophenon und 14,2 Teile Methyl-5-methylhex-5-enoat werden bei Raumtemperatur zu einer Lösung aus 32 Teilen Methanol und 100 Teilen Schwefelsäure gegeben. Man rührt 3 Tage bei Raumtemperatur, giesst die Reaktionslösung anschliessend in Wasser und extrahiert die organische Phase mit Ether. Der Extrakt wird mit Natriumbicarbonatlösung und Wasser gewaschen und anschliessend eingeengt. Der Rückstand wird destilliert und man erhält eine Mischung aus 2-Hydroxy-3-(5-methoxycarbonyl-2-methylpent-2-yl)-acetophenon und 2-Hydroxy-5-(5-methoxycarbonyl-2-methylpent-2-yl)-acetophenon.

Analyse: C H
berechnet 69,04 7,97
gefunden 69,31 8,19

**Beispiel 66:** Das Verfahren von Beispiel 65 wird wiederholt mit der Abänderung, dass 2-Hydroxybenzophenon anstelle von 2-Hydroxyaceto-phenon eingesetzt wird und dass die Reaktionsdauer 5 Tage beträgt. Durch Destillation erhält man ein Gemisch von 2-Hydroxy-3-(5-methoxy-carbonyl-2-methylpent-2-yl)-benzophenon und 2-Hydroxy-5-(5-methoxy-carbonyl-2-methylpent-2-yl)-benzophenon.

Analyse: C H
berechnet 74,09 7,11
gefunden 74,00 7,32

**Beispiel 67:** Methyl-5-(4-hydroxy-3-methylphenyl)-5-methylhexanoat (Beispiel 9) wird mit Allylalkohol umgeestert. Als Katalysator wird p-Toluolsulfonsäure verwendet. Man erhält Allyl-5-(4-hydroxy-3-methyl-phenyl)-5-methylhexanoat (K.P.$_{0,7}$ 182°C bis 188°C).

Analyse: C H
berechnet 73,88 8,75
gefunden 73,69 8,66

**Beispiel 68:** Das Verfahren von Beispiel 67 wird verwendet um Cyclohexyl-5-(4-hydroxy-3-methylphenyl)-5-methylhexanoat herzustellen. Als Ausgangsprodukte dienen Cyclohexanol und Methyl-5-(4-hydroxy-3-methylphenyl)-5-metbylhexanoat. Das Produkt siedet bei $T_{0,9}$ 200°C bis 204°C.

Analyse: C H
berechnet 75,43 9,50
gefunden 75,03 9,41

**Beispiel 69:** Zu einer Suspension von 2,0 Teilen Lithiumaluminiumhydrid in 100 Teilen trockenem Tetrahydrofuran werden unter Rühren 5,3 Teile 2-Acetamido-6-(4-hydroxyphenyl)-6-methylheptan in 50 Teilen trockenen Tetrahydrofuran zugetropft. Nach Beendigung der Addition wird dis Reaktionsmischung weitere 20 Stunden bei Raumtemperatur gerührt. Danach wird Ethylacetat zugegeben um überschüssiges Lithiumaluminiumhydrid zu zerstören. Die Reaktionsmischung wird in Wasser gegossen und die festen Rückstände abfiltriert. Die wässrige Phase und der feste Rückstand wir mit Ether extrahiert und die vereinigten Etherphasen mit Wasser gewaschen. Nach dem Abdampfen des Ethers wird das verbleibende Oel in einer Kurzwegkolonne destilliert. Man erhält 2-N-Fthylamino-6-(4-hydroxyphenyl)-6-methylheptan als farbloses Oel bei 0,7 mb und einer Ofentemperatur von 190°C.

Analyse: C H N
berechnet 77,06 10,91 5,62
gefunden 76,78 11,26 5,50

**Beispiel 70:** 10,0 Teile 5-(4-Hydroxyphenyl)-5-methylhexansäure werden in 75 Teilen Toluol suspendiert und 10,0 Teile Thionylchlorid zugegeben. Die Mischung wird allmählich auf 50°C erwärmt bis die Säure gelöst ist und dann über Nacht stehen gelassen. Toluol und andere flüchtige Bestandsteile werden bei Raumtemperatur unter Vakuum abgezogen und der Rückstand mit 75 Teilen wässrigem Ammoniak (Dichte: 0,88 g/cm$^3$) behandelt. Die organische Phase wird mit Ether extrahiert und die etherische Lösungen mit verdünnter Salzsäure und anschliessend mit Wasser gewaschen. Der Ether wird verdampft und der Rückstand mit verdünnter Natronlauge aufgenommen. Zu dieser Lösung gibt man festes Kohlendioxid und erhält 5-(4-Hydroxyphenyl)-5-methylhexansäure-amid), das aus Methanol mkristallisiert wird und zwischen 137°C und 139°C schmilzt.

Analyse: C H N
berechnet 70,56 8,65 6,33
gefunden 70,48 8,57 6,03

**Beispiel 71:** 5,0 Teile Methyl-5-(4-hydroxyphenyl)-5-methylhexanoat und 50 Teile n-Butylamin werden in ein Glasrohr eingeschmolzen und 24 Stunden auf 200°C erhitzt. Nach dem Abkühlen wird überschüssiges n-Butylamin abgezogen und das zurückbleibende Oel einer Kurzwegdestillation unterworfen. Man erhält bei 0,7 mb N-n-Butyl-5-(4-hydroxyphenyl)-5-methylhexansäureamid,

Analyse: C H N
berechnet 73,61 9,81 5,05
gefunden 73,47 9,99 4,82

**Beispiel 72:** Das Verfahren von Beispiel 71 wird wiederholt, indem man 50 Teile Diethylamin an der Stelle von n-Butylamin verwendet. Die Reaktionsdauer beträgt in diesem Fall 48 Stunden. Man erhält N,N-Diethyl-5-(4-hydroxyphenyl)-5-methylhexansäureamid,

Analyse: C H N
berechnet 73,61 9,81 5,05
gefunden 73,43 9,95 4,62

**Beispiele 73 bis 75:**

Die korrosionsverhinderdernde Wirkung von Substanzen der Formel I wird im nachstehend beschriebenen Test nachgewiesen, bei dem korrodierendes Wasser folgender Zusammensetzung verwendet wird:

204,6 $dm^3$ destilliertesWasser (= 45 UK Gal.)
20 g $CaSO_4 \times 2H_2O$
15 g $MgSO_4 \times 7H_2O$
4,6 g $NaHCO_3$
7,7 g $CaCl_2 \times 6H_2O$

5 x 2,5 $cm^2$ grosse Stücke aus Flusstahl (weicher Stahl) werden mit Bimsstein abgerieben, eine Minute in Salzsäure getaucht, anschliessend abgespült, getrocknet und gewogen.

Man stellt eine Lösung von 100 ppm einer Substanz der Formel 1 in korrodierendem Wasser her, indem man 0,1 g der betreffenden Verbindung in 1000 ml Wasser der oben definierten Zusammensetzung löst. Das Stahlplättchen wird in diese Lösung eingetaucht und das System wird in einem thermostatisierten Gefäss bei 40°C belassen. Während der Lagerung wird Luft mit einer Geschwindigkeit von 500 ml/min durch die Lösung geleitet, wobei der direkte Kontakt zwischen Stahlplättchen und Luftstrom vermieden wird, und wobei die Verdampfungsverluste durch Zugabe von destilliertem Wasser ausgeglichen werden. Nach 48 Stunden wird das Stahlplättchen herausgenommen, mit Bimsstein abgerieben, eine Minute in Salzsäure getaucht, die mit 1 Gew.% Hexamethylendiamin inhibiert ist, anschliessend abgespült, getrocknet und gewogen. Man stellt einen Gewichtsverlust fest.

Zu jeder Testserie wird eine Blindversuch durchgeführt, d.h., das Stahlplättchen wird einer Behandlung im Wasser der oben definierten Zusammensetzung unterworfen, das keinen Korrosionsinhibitor der Formel I enthält.

Der Korrosionsgrad wird als mg Gewichtsverlust pro Quadratdezimeter Oberfläche und Tag berechnet. Aus Gründen einer übersichtlicheren Darstellung werden die Resultate als prozentmässige Schutzwirkung angegeben und nach der folgenden Gleichung berechnet:

$$\%\ \text{Schutzwirkung} = \frac{\text{Korrosionsgrad ohne Inhibitor} - \text{Korrosionsgrad mit Inhibitor}}{\text{Korrosionsgrad ohne Inhibitor}} \times 100$$

Die Testergebnisse sind in Tabelle 1 dargestellt.

**Tabelle 1:** Korrosionsschutz an Stahlplättchen in standardisiertem korrodierendem Wasser hervorgerufen durch Substanzen der Formel I

| Beispiel | Inhibitorsubstanz | Korrosionsschutz hervorgerufen durch 100 ppm Inhibitorsubstanz (% Schutzwirkung) |
|---|---|---|
| 73 | Produkt von Beispiel 21 b) | 98 |
| 74 | Dicarbonsäure von Beispiel 8 | 98 |
| 75 | Tricarbonsäure von Beispiel 22 | 100 |

Die Testresultate in der oberen Tabelle demonstrieren die effektive Schutzwirkung von Substanzen der Formel I.

**Beispiele 76 bis 80:**

Die Korrosionsschutzwirkung von Verbindungen der Formel I in wässrigen Schneidölen wird im folgenden Test gezeigt, der eine Modifikation des "Institute of Petroleum Test 287" (IP 287) darstellt.

Man stellt eine 1%ige wässrige Lösung des zu testenden Korrosionsinhibitors her und stellt den pH-Wert mit Triethanolamin (TEA) auf den Wert 9 ein.

Diese Lösung wird zwei-, vier-, acht- und sechzehnfach verdünnt.

In jede dieser Lösungen gibt man Gusseisenspäne wie es in der IP 287 Testprozedur vorgeschrieben ist.

Für den Test wird deionisiertes Wasser verwendet. Nach der Behandlung wird der Zustand der Metallspäne nach folgenden Richtlinien visuell beurteilt:

**Ausmass der Rostbildung Beurteilung**

kein Rost 0
weniger als 5 kleine Punkte (S)pur
weniger als 10% der Fläche Rost (M)ässig
mehr als 10% der Fläche Rost (A)usgedehnt

Die Testergebnisse sind in Tabelle 2 dargestellt. Sie zeigen deutlich die Korrosionsverhindernde Wirkung von Substanzen der Formel I inwässrigen Schneidflüssigkeiten.

**Tabelle 2:** Korrosionsschutz durch Verbindungen der Formel I in wässrigen Schneidölen getestet an Gusseisenspänen

FIG32/260

| Beispiel | Korrosionsinhibitor | % TEA bezogen auf 1% der Verbindung | pH | IP 287 Test | | |
|---|---|---|---|---|---|---|
| | | | | Verdünnung | | Rostbildung (Deionisiertes Wasser) |
| | | | | *Verhält-nis | % | |
| 76 | Produkt von Beispiel 16 | 4.8 | 9.1 | 1:68<br>1:136 | 0.25<br>1.125 | S<br>A |
| 77 | Dicarbonsäure von Beispiel 8 | 5.2 | 9.0 | 1:64<br>1:128<br>1:256 | 0.25<br>0.125<br>0.062 | 0<br>0<br>0 |
| 78 | Tricarbonsäure von Beispiel 22 | 3.6 | 9.1 | 1:22<br>1:44<br>1:88<br>1:180<br>1:360 | 1.0<br>0.5<br>0.25<br>0.125<br>0.062 | 0<br>0<br>0<br>0<br>S-M |
| 79 | Produkt von Beispiel 23 b) | 5.8 | 9.0 | 1:58<br>1:116<br>1:232 | 0.25<br>0.125<br>0.062 | 0<br>M<br>A |
| 80 | Produkt von Beispiel 26 b) | 3.9 | 9.0 | 1:82<br>1:164 | 0.25<br>0.125 | 0<br>A |

*Das Verdünnungsverhältnis bezieht sich auf die Anteile (TEA + Verbindung der Formel I):Wasser
Die %-Angabe bezieht sich auf die Menge der Verbindung der Formel I in der Testlösung

**Beispiel 81:** Die Aktivität einer Verbindung der Formel I als thermisches Antioxidans in Schmierölen wird mit Hilfe des "Institutes of Petroleum Test 229" nachgewiesen. Das Testsystem bestehend aus Oel, Wasser und Kupferspule als Katalysator befindet sich in einem bedeckten Glasgefäss und wird in einen Autoklaven, der mit einem Manometer ausgerüstet ist, gestellt. Das System wird mit Sauerstoff unter einen Druck von 6,4 bar gesetzt, in ein Oelbad von 150°C gestellt und mit 100 Umdrehungen/min um eine Achse, die 30° von der Horizontalen abweicht, rotiert. Da das Oel mit dem Sauerstoff reagiert, sinkt der Innendruck ab. Es wird die Zeit gemessen, nach der ein bestimmter Innendruck erreicht wurde.

Die Messdaten in Tabelle 3 zeigen, dass Substanzen der Formel I die Oxidation von Rotationspumpenöl (RVO) inhibieren und dass die Bildung saurer Zersetzungsprodukte des Oels reduziert wird, wenn man mit einem Oel ohne Additiv der Formel I vergleicht.

**Tabelle 3: Verringerung der Oxidationsgeschwindigkeit eines Schmieröls durch Verwendung von Verbindungen der Formel I als Stabilisatoren**

| Beispiel | Verbindung der Formel I | Säurezahl (mg KOH/g) | Standardisierte Lebensdauer (min) |
|---|---|---|---|
| Vergleich | RVO ohne Additiv | 2,4 | 58 |
| 81 | RVO enthaltend Produkt aus Beispiel 57 | 0,28 | 228 |

**Patentansprüche:**

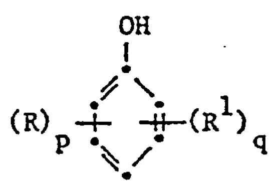

(I) ,

worin p 1,2 oder 3 ist, q 0, 1 oder 2 ist, mit der Massgabe, dass $(p+q) \leqslant 3$ ist, und worin R eine Gruppe der Formel II bedeutet

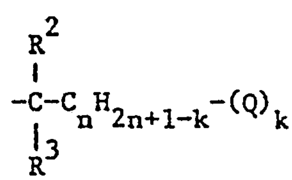

(II) ,

wobei die Gruppen R im Fall von p = 2 oder p = 3 gleich oder innerhalb der gegebenen Definitionen verschieden sind und sich in 2-, 4- oder 6-Position befinden, und n eine ganze Zahl von 2 und 30 ist, und k 1 oder 2 bedeutet, und schliesslich Q einer der folgenden Reste ist:

a) $-COOR^4$ oder $-CONR^4R^5$, wobei $R^4$ Wasserstoff, geradkettigesoder verzweigtes $C_1$-$C_{20}$Alkyl, das gegebenenfalls mit 1 bis 5 Sauerstoffatomen unterbrochen ist oder gegebenenfalls Substituenten $-OR^6$ trägt, wobei $R^6$ $C_3$-$C_{12}$ Cycloalkyl, geradkettiges oder verzweigtes $C_3$-$C_{20}$ Alkenyl oder $C_6$-$C_{10}$ Aryl bedeutet, das gegebenenfalls mit ein oder zwei $C_1$-$C_4$-Alkylgruppen substituiert ist, oder $C_7$-$C_{13}$ Aralkyl ist, und wo $R^4$ zusätzlich divalentes geradkettiges oder verzweigtes $C_2$-$C_{20}$ Alkylen ist, oder geradkettiges oder verzweigtes $C_3$-$C_{20}$ Alkenyl bedeutet, oder $C_3$-$C_{12}$ Cycloalkyl oder $C_6$-$C_{10}$ Aryl bedeutet, das gegebenenfalls mit einer $C_1$-$C_4$ Alkylgruppe substituiert ist, oder $C_7$-$C_{13}$ Aralkyl ist, oder ein fünf- oder sechsgliedriger sauerstoffhaltiger Heterozyklus ist, der gegebenenfalls mit ein oder zwei geradkettigen oder verzweigten $C_1$-$C_4$Alkylgruppen substituiert ist, oder wo $R^4$ Methyl bedeutet, das durch einen fünf- oder sechsgliedrigen sauerstoffhaltigen gegebenenfalls ein oder zwei $C_1$-$C_4$Alkylgruppen enthaltenden Heterozyklus substituiert ist, mit der Massgabe, dass die beiden C-Atome, an denen sich die $-COOR^4$ Gruppen im Fall von $k = 2$ befinden, nicht benachbart sind, und wobei $R^5$ Wasserstoff oder geradkettiges oder verzweigtes $C_1$-$C_{20}$Alkyl ist, oder wo $R^4$ und $R^5$ zusammen mit dem gemeinsamen Stickstoffatom einen fünf- oder sechsgliedrigen heterozyklischen Ring bilden, der gegebenenfalls mit ein oder zwei $C_1$-$C_4$Alkylgruppen substituiert sein kann,

b) -OX, wobei X die gleiche Bedeutung wie $R^5$ hat, oder $-COR^7$, wobei $R^7$ Wasserstoff, geradkettiges oder verzweigtes $C_1$-$C_{20}$Alkyl, geradkettiges oder verzweigtes $C_3$-$C_{20}$Alkenyl,$C_3$-$C_{12}$Cycloalkyl, $C_7$-$C_{13}$Aralkyl oder $C_6$-$C_{10}$Aryl ist, das gegebenenfalls mit ein oder zwei $C_1$-$C_4$Alkylgruppen substituiert sein kann,

c) $-NR^8R^9$, wobei $R^8$ Wasserstoff oder geradkettiges oder verzweigtes $C_1$-$C_4$Alkyl ist, und $R^9$ Wasserstoff, geradkettiges oder verzweigtes $C_1$-$C_4$Alkyl, oder $-COR^7$ ist, wobei $R^7$ die früher definierte Bedeutung hat, oder wobei $R^8$ und $R^9$ zusammen mit dem gemeinsamen N-Atom einen fünf- oder sechsgliedrigen Ring bilden, welcher gegebenenfalls mit ein oder zwei $C_1$-$C_4$Alkylgruppen substituiert ist,

d) $-PO(OR^{10})[O]_x-R^{11}$, wobei x 0 oder 1 ist, und wobei $R^{10}$ und $R^{11}$ im Falle von $x = 1$ gleich oder verschieden sind und unabhängig voneinander Wasserstoff, geradkettiges oder verzweigtes $C_1$-$C_{20}$Alkyl bedeuten oder wobei $R^{10}$ und $R^{11}$ eine zusammenhängende $C_2$-$C_3$Alkylen-kette, die gegebenenfalls mit einer oder mehreren $C_1$-$C_{20}$Alkylgruppen substituiert sein kann, bilden, oder wobei im Fall $x = 0$ $R^{10}$ Wasserstoff oder geradkettiges oder verzweigtes $C_1$-$C_{20}$Alkyl ist, und $R^{11}$ geradkettiges $C_1$-$C_5$Alkyl ist,

e) -CN, Halogen, $-NO_2$ oder

f) $-COR^{12}$, wobei $R^{12}$ Wasserstoff, geradkettiges oder verzweigtes $C_1$-$C_{20}$Alkyl oder Halogen bedeutet;

und wo in Formel II $R^2$ und $R^3$ gleich oder innerhalb der gegebenen Definition verschieden sind und geradkettiges oder verzweigtes $C_1$-$C_5$-Alkyl bedeuten, oder wo $R^2$ oder $R^3$ gegebenenfalls mit einer $-COOR^4$ Gruppe substituiert ist, aber nur dann,wenn $Q = -COOR^4$ ist, wobei die Reste $R^4$ voneinander unabhängig sind, oder wo $R^2$ oder $R^3$ so mit dem Rest $C_nH_{2n+1-k}$ verbunden sind, dass ein $C_5$-$C_{12}$Cycloalkylenrest entsteht, der mit $-(CO_2R^4)_k$ Gruppen substituiert ist, wobei die Reste$R^4$ voneinander unabhängig sind und wo R4 und k die oben definierte Bedeutung haben, oderwo $R^2$ oder $R^3$ so mit dem Rest $C_nH_{2n+1-k}$ verbunden sind, dass im Fall, wo $Q = -CO_2R^{12}$ ist, ein $C_5$-$C_{12}$Cycloalkylenrest entsteht, der gegebenenfalls mit einer $-COR^{12}$ Gruppe substituiert ist; und wobei im Fall, dass $Q = -COOR^4$ und $R^4 = C_2$-$C_{20}$ Alkylen sind, p und k 1 bedeuten, und die Verbindung durch die Formel Ia dargestellt wird

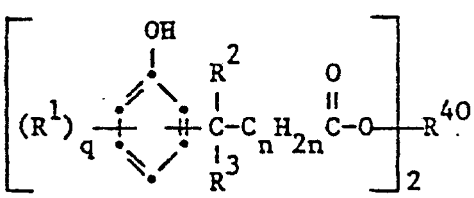

(Ia) ,

wobei $R^{40}$ eine divalente geradkettige oder verzweigte $C_2$-$C_{20}$Alkylen-kette ist;

und wobei im Fall, dass $Q = -CONR^4R^5$ und $R^4 = C_2$-$C_{20}$Alkylen sind, p und k 1 bedeuten, und die Verbindung durch die Formel Ib dargestellt wird

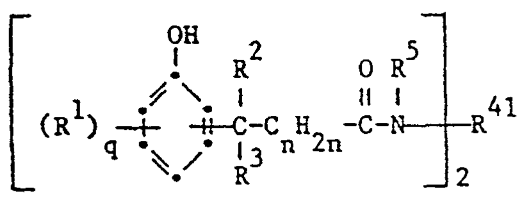 (Ib) ,

wobei $R^{41}$ ein geradkettiger oder verzweigter divalenter $C_2$-$C_{20}$Alkylenrest ist;

und wo zurückgehend auf Formel I $R^1$ geradkettiges oder verzweigtes $C_1$-$C_{12}$Alkyl, $C_7$-$C_9$Aralkyl, Halogen, $CF_3$, SH, $SR^{13}$, COOH, $COOR^{13}$, $COR^{13}$, $COC_6H_5$,$CONH_2$, CN, $SO_3H$, $SO_2NH_2$, $PO(OH)_2$, $PO(OR^{13})$, oder $NO_2$ bedeutet, wobei $R^{13}$ $C_1$-$C_4$Alkyl ist, wobei im Fall q = 2 die Gruppen $R^1$ gleich oder im Rahmen der gegebenen Definitionen unterschiedlich sein können, und wobei die Gruppe $R^1$ im Fall q = 1 zusätzlich ein Rest der Formel III oder IV sein kann

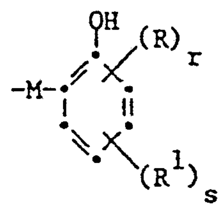 (III) , 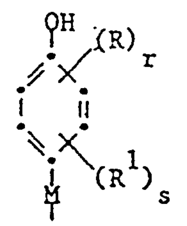 (IV) ,

worin R und $R^1$ die oben definierte Bedeutung besitzen, r 0, 1 oder 2 ist, s 0 oder 1 bedeutet mit der Massgabe, das $(r+s) \leqslant 2$ ist, und wobei M eine direkte Bindung, $-(R^{14})C(R^{15})-$, -S-, -S-S-, -SO-, $-SO_2-$, $-CH_2SCH_2-$, -O-, $-CH_2OCH_2-$ oder $-NR^{16}-$ ist, wobei $R^{14}$und $R^{15}$ unabhängig voneinander Wasserstoff, geradkettiges oder verzweigtes $C_1$-$C_{20}$-Alkyl, das gegebenenfalls durch 1 bis 3 Schwefelatome unterbrochen sein kann, oder $C_6$-$C_{10}$Aryl sein können, oder wobei $R^{14}$ und $R^{15}$ zusammen mit dem gemeinsamen C-Atom einen fünf- oder sechsgliedrigen Ring bilden, der gegebenenfalls mit ein oder zwei $C_1$-$C_8$Alkylgruppen substituiert sein kann, und wobei $R^{16}$ Wasserstoff, geradkettiges oder verzweigtes $C_1$-$C_{20}$Alkyl oder Phenyl ist; wobei die Verbindung der Formel I nur einen Rest der Formel III oder IV enthält, und wobei dieser. Rest in 2-,4- oder 6-Position zur Hydroxygruppe der Formel I steht, und wobei im Fall, dass p = 1, $R^1 = C_1$-$C_{12}$Alkyl, $R^2$ und $R^3$ $= C_1$-$C_5$Alkyl, k = 1, Q=$-COOR^4$ und $R^4$=Wasserstoff sind, n nicht 2 ist; zusätzlich betrifft die Erfindung Salze der Verbindungen der Formel I mit organischen oder anorganischen Säuren und Basen.

2. Verbindungen gemäss Anspruch 1 der Formel V

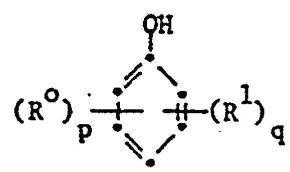 (V) ,

wobei $R^1$, p und q die gleiche Bedeutung wie in Anspruch 1 definiert besitzen, und wobei R° ein Rest der Formel IIa oder IIb ist

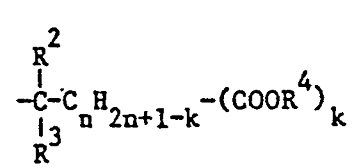 (IIa), 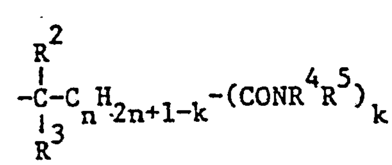 (IIb),

worin n, k, $R^2$, $R^3$, $R^4$ und $R^5$ die gleiche Bedeutung wie in Anspruch 1 besitzen.

3. Verbindungen der Formel V gemäss Anspruch 2, worin k 1 ist.

4. Verbindungen der Formel V gemäss Anspruch 2, die einen Rest der Formel IIa enthalten.

5. Verbindungen der Formel V gemäss Anspruch 4, bei denen $R^2$ und $R^3$ Methyl bedeuten und bei denen $R^1$ geradkettiges oder verzweigtes $C_1$-$C_5$Alkyl ist.

6. Verbindungen der Formel V gemäss Anspruch 2 mit n = 3.

7. Verbindungen der Formel I gemäss Anspruch 1, bei denen p = 1 ist.

8. Verbindungen der Formel I gemäss Anspruch 7 mit k = 1.

9. Verbindungen der Formel I gemäss Anspruch 8, bei denen $R^2$ und $R^3$ Methyl sind und $R^1$ geradkettiges oder verzweigtes $C_1$-$C_5$Alkyl ist.

10. Verbindungen der Formel I gemäss Anspruch 1 mit n von 3 bis 10.

11. Verbindungen der Formel V gemäss Anspruch 2, die weder einen Rest der Formel III noch einen Res der Formel IV tragen.

12. Verfahren zur Herstellung der Verbindungen der Formel I gemäss Anspruch 1, dadurch gekennzeichnet, dass ein Phenol der Formel VI in Gegenwart eines geeigneten Katalysators mit einem Alkylierungsreagenz unter Einführung einer Gruppe der Formel II umgesetzt wird

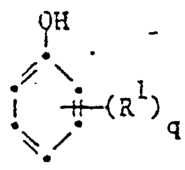 (VI) , 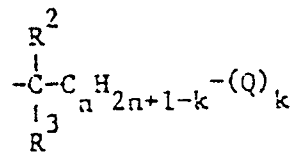 (II) ,

worin $R^1$, $R^2$, $R^3$, Q, n, q und k die in Anspruch I definierte Bedeutung besitzen, und worin die Verbindung VI nur einen der Reste III oder IV in 2-, 4- oder 6-Position zur Hydroxygruppe trägt.

13. Zusammensetzungen enthaltend ein Substrat und als Stabilisator wenigstens eine Verbindung der Formel I gemäss Anspruch I.

14. Zusammensetzung gemäss Anspruch 13, bei der als Substrat ein Zweiphasensystem vorliegt, bestehend aus einem wässrigen Medium in Kontakt zu Metallen.

15. Zusammensetzung gemäss Anspruch 14, bei der als Metalle Eisenmetalle verwendet werden.

16. Zusammensetzungen gemäss Anspruch 13, bei der das Substrat ein oxidationsempfindliches organisches Material ist.

**Claims:**

1. A compound of formula I

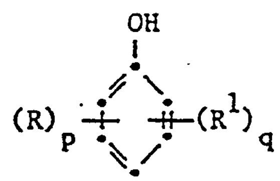 (I)

wherein p is 1, 2 or 3, q is 0, 1 or 2, with the proviso that $(p+q) \leqq 3$, and wherein R is a group of formula II

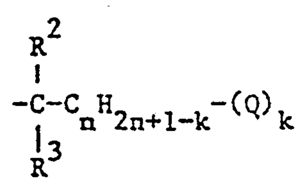 (II)

and, when p is 2 or 3, the groups R are the same or, within the scope of the above definitions, different and are in the 2-, 4- or 6-positions, and n is an integer from 2 to 30, and k is 1 or 2, and Q is selected from the radicals:

a) -$COOR^4$ or -$CONR^4R^5$, wherein $R^4$ is hydrogen; straight chain or branched $C_1$-$C_{20}$alkyl which may be interrupted by 1 to 5 oxygen atoms and is unsubstituted or substituted by -$OR^6$ groups, wherein $R^6$ is $C_3$-$C_{12}$cycloalkyl, straight chain or branched $C_3$-$C_{20}$alkenyl, $C_6$-$C_{10}$aryl which is unsubstituted or substituted by one or two $C_1$-$C_4$alkyl groups, or is $C_7$-$C_{13}$aralkyl; and $R_4$ is additionally divalent straight chain or branched $C_2$-$C_{20}$-

alkylene; straight chain or branched $C_3$-$C_{20}$alkenyl; $C_3$-$C_{12}$-cycloalkyl; $C_6$-$C_{10}$aryl which is unsubstituted or substituted by a $C_1$-$C_4$alkyl group; or is $C_7$-$C_{13}$aralkyl; or is a 5- or 6-membered oxygen-containing heterocyclic ring which is unsubstituted or substituted by one or two straight chain or branched $C_1$-$C_4$alkyl groups; or $R_4$ is methyl which is unsubstituted or substituted by a 5- or 6-membered oxygen-containing heterocyclic ring which is unsubstituted or substituted by one or two $C_1$-$C_4$alkyl groups, with the proviso that, when k is 2, the two carbon atoms to which the -$COOR^4$ groups are attached are not adjacent; and $R^5$ is hydrogen or straight chain or branched $C_1$-$C_{20}$alkyl; or $R^4$ and $R^5$, together with the nitrogen atom to which they are attached, form a 5- or 6-membered heterocyclic ring which is unsubstituted or substituted by one or two $C_1$-$C_4$alkyl groups;

b) -OX, wherein X has the same meaning as $R^5$, or is -$COR^7$, wherein $R^7$ is hydrogen, straight chain or branched $C_1$-$C_{20}$-alkyl, straight chain or branched $C_3$-$C_{20}$alkenyl, $C_3$-$C_{12}$-cycloalkyl, $C_7$-$C_{13}$aralkyl, or is $C_6$-$C_{10}$aryl which may be unsubstituted or substituted by one or two $C_1$-$C_4$alkyl groups;

c) -$NR^8R^9$, wherein $R^8$ is hydrogen or straight chain or branched $C_1$-$C_4$alkyl, and $R^9$ is hydrogen, straight chain or branched $C_1$-$C_4$alkyl, or is -$COR^7$, wherein $R^7$ is as defined above, or $R^8$ and $R^9$, together with the nitrogen atom to which they are attached, form a 5- or 6-membered ring which is unsubstituted or substituted by one or two $C_1$-$C_4$alkyl groups;

d) -$PO(OR^{10})[O]_x$-$R^{11}$, wherein x is 0 or 1, and, when x is 1, $R^{10}$ and $R^{11}$ are the same or different and are each independently hydrogen or straight chain or branched $C_1$-$C_{20}$alkyl, or $R^{10}$ and $R^{11}$ are linked together to form a $C_2$-$C_3$alkylene chain which may be unsubstituted or substituted by one or more $C_1$-$C_{20}$alkyl groups, or, when x is 0, $R^{10}$ is hydrogen or straight chain or branched $C_1$-$C_{20}$alkyl, and $R^{11}$ is straight chain $C_1$-$C_5$alkyl;

e) -CN, halogen or -$NO_2$; or

f) -$COR^{12}$, wherein $R^{12}$ is hydrogen, straight chain or branched $C_1$-$C_{20}$alkyl or halogen;

and $R^2$ and $R^3$ in formula II are the same or, within the scope of the above definitions, different and are straight chain or branched $C_1$-$C_5$alkyl, or $R^2$ or $R^3$ may be substituted by a -$COOR^4$ group, but only if Q is -$COOR^4$, the radicals $R^4$ being independent of one another, or $R^2$ or $R^3$ is so linked to the radical $C_nH_{2n+1-k}$ as to form a $C_5$-$C_{12}$cycloalkylene radical which is substituted by -$(CO_2R^4)_k$ groups, the radicals $R^4$ being independent of one another and $R^4$ and k being as defined above, or $R^2$ or $R^3$ is so linked to the radical -$C_nH_{2n+1-k}$ as to form, when Q is $COR^{12}$, a $C_5$-$C_{12}$-cycloalkylene radical which may be substituted by a -$COR^{12}$ group, and, when Q is -$COOR^4$ and $R^4$ is $C_2$-$C_{20}$-alkylene, then p and k are 1, and the compound is of formula Ia

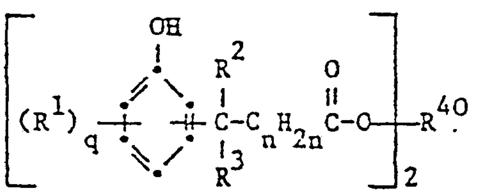

(Ia)

wherein $R^{40}$ is a divalent straight chain or branched $C_2$-$C_{20}$-alkylene chain;

and, when Q is -$CONR^4R^5$ and $R^4$ is $C_2$-$C_{20}$alkylene, then p and k are 1, and the compound is of formula Ib

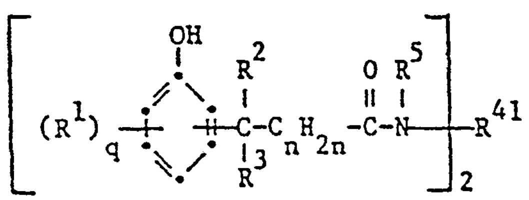

(Ib)

wherein $R^{41}$ is a straight chain or branched $C_2$-$C_{20}$alkylene chain;

and in which formulae, reverting to formula I, $R^1$ is straight chain or branched $C_1$-$C_{12}$alkyl, $C_7$-$C_9$aralkyl, halogen, $CF_3$, SH, $SR^{13}$, COOH, $COOR^{13}$, $COR^{13}$, $COC_6H_5$, $CONH_2$, CN, $SO_3H$, $SO_2NH_2$, $PO(OH)_2$, $PO(OR^{13})$ or $NO_2$, with $R^{13}$ being $C_1$-$C_4$alkyl, and, when q is 2, the groups $R^1$ may be the same or, within the scope of the above definitions, different, and, when q is 1, the group $R^1$ may additionally be a radical of formula III or IV

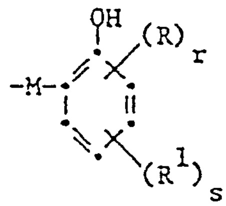 (III) 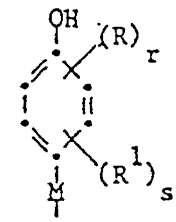 (IV)

wherein R and $R^1$ are as defined above, r is 0, 1 or 2, s is 0 or 1, with the proviso that $(r+s) \leq 2$, and wherein M is a direct bond, $-(R^{14})C(R^{15})-$, -S-, -S-S- -SO-, $-SO_2-$, $-CH_2SCH_2-$, -O-, $-CH_2OCH_2-$ or $-NR^{16}-$, wherein each of $R^{14}$ and $R^{15}$ independently of the other may be hydrogen or straight chain or branched $C_1$-$C_{20}$alkyl which may be interrupted by 1 to 3 sulfur atoms, or may be $C_6$-$C_{10}$aryl, or $R^{14}$ and $R^{15}$, together with the carbon atom to which they are attached, form a 5- or 6-membered ring which may be unsubstituted or substituted by one or two $C_1$-$C_8$alkyl groups, and $R^{16}$ is hydrogen, straight chain or branched $C_1$-$C_{20}$alkyl, or is phenyl; with the proviso that the compound of formula I contains only one radical of the formula III or IV, and that this radical is in the 2-, 4- or 6-position relative to the hydroxyl group of formula I, and, when p is 1, $R^1$ is $C_1$-$C_{12}$alkyl, $R^2$ and $R^3$ are $C_1$-$C_5$alkyl, k is 1, Q is $-COOR^4$ and $R^4$ is hydrogen, and n is not 2;

or a salt of a compound of formula I with an organic or inorganic acid or base.

2. A compound according to claim 1 of formula V

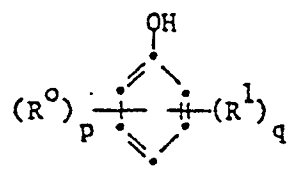 (V)

wherein $R^1$, p and q are as defined in claim 1, and R° is a radical of formula IIa or IIb

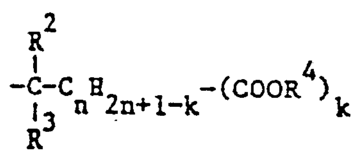 (IIa) 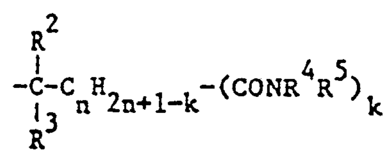 (IIb)

wherein n, k, $R^2$, $R^3$, $R^4$ and $R^5$ are as defined in claim 1.

3. A compound of formula V according to claim 2, wherein k is 1.

4. A compound of formula V according to claim 2, which contains a radical of formula IIa.

5. A compound of formula V according to claim 4, wherein $R^2$ and $R^3$ are methyl and $R^1$ is straight chain or branched $C_1$-$C_5$alkyl.

6. A compound of formula V according to claim 2, wherein n is 3.

7. A compound of formula I according to claim 1, wherein p is 1.

8. A compound of formula I according to claim 7, wherein k is 1.

9. A compound of formula I according to claim 8, wherein $R^2$ and $R^3$ are methyl and $R^1$ is straight chain or branched $C_1$-$C_5$alkyl.

10. A compound of formula I according to claim 1, wherein n is from 3 to 10.

11. A compound of formula V according to claim 2, which contains neither a radical of formula III nor a radical of formula IV.

12. A process for the preparation of a compound of formula I according to claim 1, which process comprises reacting a phenol of formula VI, in the presence of a suitable catalyst, with an alkylating agent to introduce a group of formula II

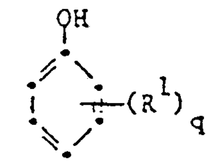

(VI)

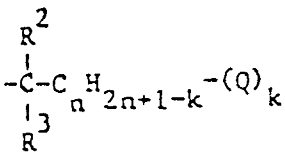

(II)

wherein $R^1$, $R^2$, $R^3$, Q, n, q and k are as defined in claim 1, withthe proviso that the compound VI contains only one of the radicals III or IV in the 2-, 4- or 6-position relative to the hydroxyl group.

13. A composition containing a substrate and, as stabiliser, at least one compound of formula I according to claim 1.

14. A composition according to claim 13, wherein the substrate is a two-phase system comprising an aqueous medium in contact with metals.

15. A composition according to claim 14, wherein the metals are ferrous metals.

16. A composition according to claim 13, wherein the substrate is an organic material susceptible to oxidative decomposition.

**Revendications**

1. Composés de formule I

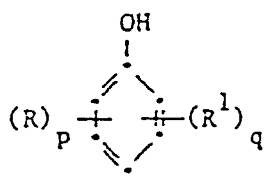

(I) ,

dans laquelle p est 1, 2 ou 3, Q est 0,1 ou 2, dans la mesure où $(p + q) \leqq 3$, et dans laquelle R représente un groupe de formule II

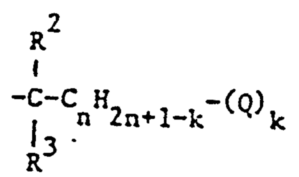

(II) ,

auquel cas les groupes R, dans le cas de p = 2 ou p = 3, sont identiques ou différents dans le cadre des définitions données et se trouvent en position -2, -4, ou -6, n est un nombre entier de 2 et 30, k représente 1 ou 2 et finalement Q est un des restes suivants:

a) $-COOR^4$> ou $-CONR^4R^5$, auquel cas $R^4$ est l'hydrogène, un alkyle à chaîne droite ou ramifiée en $C_1$ à $C_{20}$ qui est éventuellement interrompu par 1 à 5 atomes d'oxygène ou éventuellement porteur de substituants $-OR^6$, $R^6$ représentant un cycloalkyle en $C_3$ à $C_{12}$, un alcényl à à chaîne droite ou ramfiée en $C_3$ à $C_{20}$ ou un aryle en $c_6$ à $C_{10}$, éventuellement substitué par un ou deux groupes alkyles en $C_1$ à $C_4$, ou un aralkyl en $C_7$ à $C_{13}$ et où $R^4$ est en outre un alkylène à chaîne droite ou ramifiée divalent en $C_2$ à $C_{20}$ ou représente un alcényle à chaîne droite ou ramifiée en $C_3$ à $C_{20}$, un cycloalkyle en $C_3$ à $C_{12}$ ou un aryle en $C_6$ à $C_{10}$ qui est éventuellement substitué par un groupe alkyl en $C_1$ à $C_4$ ou est un aralkyle en $C_7$ à $C_{13}$ ou en hétérocycle penta- ou hexagonal contenant de l'oxygène, éventuellement substitué par un ou deux groupes alkyles à chaîne droite ou ramifiée en $C_1$ à $C_4$, où $R^4$ représente un méthyle substitué par un hétérocycle penta- ou hexagonal oxygéné contenant éventuellement un ou deux groupes alkyles en $C_1$ à $C_4$, dans la mesure où les deux atomes de C sur lesquels se trouvent les groupes $-COOR^4$ dans le cas de k = 2 ne sont pas contigues et où $R^5$ est l'hydrogène ou un alkyle à chaîne droite ou

ramifiée en $C_1$ à $C_{20}$ ou $R^4$ et $R^5$ forment avec l'atome d'azote commun un noyau hétérocyclique penta- ou hexagonal pouvant être éventuellement substitué par un ou deux groupes alkyles en $C_1$ à $C_5$,

b) -OX, auguel cas X a la même signification que $R^5$, ou $-COR^7$, $R^7$ étant l'hydrogène, un alkyle à chaîne droite ou ramifiée en $C_1$ à $C_{20}$, un alcényle à chaîne droite ou ramifiée en $C_3$ à $C_{20}$, un cycloalkyle en $C_3$ à $C_{12}$, un aralkyle en $C_7$ à $C_{13}$ ou un aryle en $C_6$ à $C_{10}$ pouvant être éventuellement substitue par un ou deux groupes alkyles en $C_1$ à $C_4$,

c) $-NR^8R^9$, auquel cas $R^8$ est l'hydroqène ou un alkyle à chaîne droite ou ramifiée en $C_1$ à $C_4$ et $R^9$ est l'hydroqène, un alkyle à chaîne droite ou ramifiée en $C_1$ à $C_4$ ou $-COR^7$,$R^7$ ayant la signification précédente ou $R^8$ et $R^9$ formant avec l'atome de N commun un cycle penta- ou hexaqonal qui est éventuellement substitué par un ou deux groupes alkyles en $C_1$ a $C_4$,

d) $-PO(OR^{10})[O]_x-R^{11}$, auquel cas x est 0 ou 1 et $R^{10}$ et $R^{11}$, dans le cas de $x = 1$, sont identiques ou différents et représentent, indépendamment l'un de l'autre, l'hydroqène, un alkyle à chaîne droite ou ramifiée en $C_1$ à $C_{20}$ ou $R^{10}$ et $R^{11}$ forment une chaîne alkylene associée en $C_2$ à $C_3$ pouvant être éventuellement substituée par un ou plusieurs groupes alkyles en $C_1$ à $C_{20}$ ou, dans le cas de $x = 0$, $R^{10}$ est l'hydrogène ou un alkyle à chaîne droite ou ramifiée en $C_1$ à $C_{20}$ et $R^{11}$ est un alkyle à chaîne droite en $C_1$ à $C_5$,

e) -CN, un halogène, $-NO_2$ ou

f) $-COR^{12}$, auquel cas $R^{12}$ représente l'hydrogène, un alkyle à chaîne droite ou ramifiée en $C_1$ à $C_{20}$ ou un halogène; et où, dans la formule II, $R^2$ et $R^3$ sont identiques ou différents dans le cadre de la définition donnée et représentent un alkyle à chaîne droite ou ramifiée en $C_1$ à $C_5$ ou $R^2$ ou$R^3$ est éventuellement substitué par un groupe $-COOR^4$ mais seulement lorsque Q est $-COOR^4$, auquel cas les restes $R^4$ sont indépendants les uns des autres ou $R^2$ ou $R^3$ est lié au reste $C_nH_{2n+1-k}$ de manière qu'il se forme un reste cycloalkylène en $C_5$ à $C_{12}$ qui est substitué par des groupes $-(CO_2R^4)_k$, les restes $R^4$ étant indépendants les uns des autres, $R^4$ et k possédant la signification précédente ou $R^2$ ou $R^3$ étant lié au reste $C_nH_{2n+1-k}$ de manière qu'il se forme, dans le cas où $Q = CO_2R^{12}$ un reste cycloalkylène en $C_5$ à $C_{12}$ gui est éventuellement substitué par un groupe $-COR^{12}$; et dans le cas où $Q = COOR^4$ et $R^4$ = un alkylène en $C_2$ à $C_{20}$, p et k représentent 1 et le composé est représenté par la formule Ia

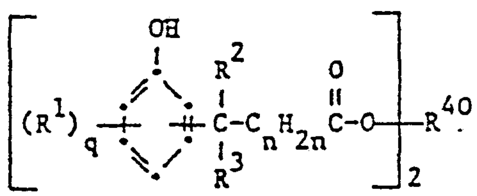

(Ia) ,

dans laguelle $R^{40}$ est une chaîne alkylène à chaîne droite ou ramifiée divalente en $C_2$ à $C_{20}$; et dans le cas où $Q = CONR^4R^5$ et $R^4$ = un alkylène en $C_2$ à $C_{20}$, p et k représentent 1 et le composé est représenté par la formule Ib

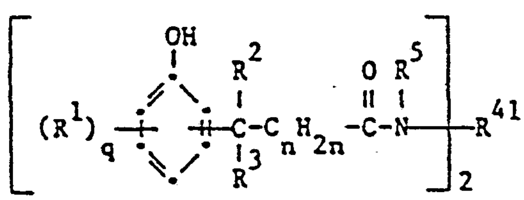

(Ib) ,

dans laquelle $R^{41}$ est un reste alkylène à chaîne droite ou ramifiée divalent en $C_2$ à $C_{20}$; et en se reportant à la formule I, $R^1$ represente un alkyle à chaîne droite ou ramifiée en $C_1$ à $C_{12}$, un aralkyle en $C_7$ à $C_9$, un halogène, $CF_3$, SH, $SR^{13}$, COOH,$COOR^{13}$, $COR^{13}$, $COC_6H_5$, $CONH_2$, CN, $SO_3H$, $SO_2NH_2$, $PO(OR^{13})$ ou $NO_2$, auquel cas $R^{13}$ est un alkyle en $C_1$ à $C_4$, moyennant quoi, dans le cas de $q = 2$, les groupes $R^1$ peuvent être identiques ou différents dans le cadre des définitions données et le groupe $R^1$, dans le cas de $q = 1$, peut en outre être un reste de formule III ou IV

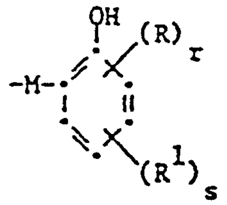 (III) , 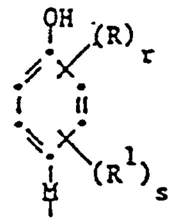 (IV) ,

dans laquelle R et $R^1$ ont la signification définie précédemment, r est 0, 1 ou 2, s représente 0 ou 1 dans la mesure où $(r+s) \leqslant 2$ et M est une liaison directe, $-(R^{14})C(R^{15})$ -S-, -S-S-, -SO-, $-SO_2-$, $-CH_2SCH_2-$, -O-, $-CH_2OCH_2-$ ou $-NR^{16}$, moyennant quoi $R^{14}$ et $R^{15}$ peuvent être, independamnent l'un de l'autre, l'hydrogène, un alkyle à chaîne droite ou ramifiée en $C_1$ à $C_{20}$ qui peut être éventuellement interrompu par 1 à 3 atomes de soufre ou un aryle en $C_6$ à $C_{10}$ ou $R^{14}$ et $R^{15}$ forment, conjointement avec l'atome de C commun, un cycle penta- ou hexagonal qui peut être éventuellement substitué par un ou deux groupes alkyles en $C_1$ à $C_8$ et $R^{16}$ est l'hydrogène, un alkyle à chaîne droite ou ramifiée en $C_1$ à $C_{20}$ ou un phényle; auquel cas le composé de formule I ne contient qu'un reste de formule III ou IV, et ce reste est placé en position -2, -4 ou -6 par rapport au groupe hydroxyle de la formule I et, dans le cas de p = 1, $R^1$ = alkyle en $C_1$ à $C_{12}$, $R^2$ et $R^3$ = alkyle en $C_1$ à $C_5$, k = 1, Q = $-COOR^4$ et $R^4$ = hydrogène, n n'est pas 2; l'invention concerne en outre des sels des composés de formule I avec des acides et des bases organiques ou inorganiques.

2. Composés selon la revendication 1, répondant à la formule V

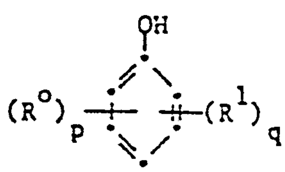 (V) ,

dans laquelle $R^1$, p et q ont la même signification que dans la revendication 1 et R° est un reste de formule IIa ou IIb

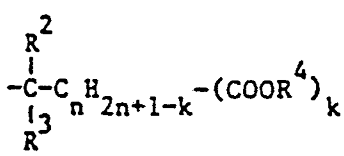 (IIa), 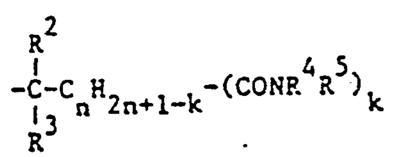 (IIb),

où n, k, $R^2$, $R^3$, $R^4$ et $R^5$ ont la même signification que dans la revendication 1.

3. Composés de formule V selon la revendication 2, dans lesquels k est 1.

4. Composés de formule V selon la revendication 2, contenant un reste de formule IIa.

5. Composés de formule V selon la revendication 4, dans lesquels $R^2$ et $R^3$ représentent des méthyles et $R^1$ est un alkyle à chaîne droite ou ramifiée en $C_1$ à $C_5$.

6. Composés de formule V selon la revendication 2 avec n = 3.

7. Composés de formule I selon la revendication 1. dans lesquels p = 1.

8. Composés de formule I selon la revendication 7 avec k = 1.

9. Composés de formule I selon la revendication 8. dans lesquels $R^2$ et $R^3$ sont les méthyles et $R^1$ est un alkyle à chaîne droite ou ramifiée en $C_1$ à $C_5$.

10. Composés de formule I selon la revendication 1, avec n s'étendant de 3 à 10.

11. Composés de formule V selon la revendication 2 qui ne sont porteurs ni d'un reste de formule III, ni d'un reste de formule IV.

12. Procédé pour la préparation de composés de formule I selon la revendication 1. caractérisé en ce que l'on fait réagir un phénol de formule VI, en présence d'un catalyseur approprié. avec un réactif d'alkylation et introduction d'un groupe de formule II

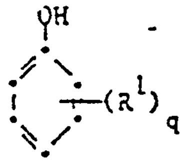 (VI) , 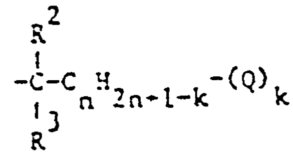 (II) ,

formules dans lesquelles $R^1$, $R^2$, $R^3$, Q, n, q et k ont la signification définie dans la revendication 1 et dans lesquelles le composé VI. n'est porteur que d'un des restes III ou IV en position -2, -4 ou -6 par rapport au groupe hydroxyle.

13. Compositions contenant un substrat et. comme stabilisant. au moins un composé de formule I selon la revendication 1.

14. Composition selon la revendication 13 dans laquelle est présent.en tànt que substrat.un système à deux phases consistant en un milieu aqueux en contact avec des métaux.

15. Composition selon la revendication 14 dans laquelle des metaux ferreux sont utilisés comme métaux.

16. Composition selon la revendication 13 dans laquelle le substrat est une matière organique sensible à l'oxydation.